# FASCICULE DE BREVET EUROPEEN

(11) **EP 0 817 779 B1**
(45) Date de publication et mention de la délivrance du brevet: **05.01.2000**
(21) Numéro de dépôt: 96909192.5
(22) Date de dépôt: 25.03.1996
(51) Int. Cl.: C07D 305/14, A61K 31/335

(54) **NOUVEAUX TAXOIDES, LEUR PREPARATION ET LES COMPOSITIONS PHARMACEUTIQUES QUI LES CONTIENNENT**
TAXAL DERIVATE, DEREN HERSTELLUNG UND DIESE ENTHALTENDE PHARMAZEUTISCHE ZUSAMMENSETZUNG
NOVEL TAXOIDS, PREPARATION THEREOF AND PHARMACEUTICAL COMPOSITIONS CONTAINING SAME

(30) Priorité: 27.03.1995 FR 9503545; 22.12.1995 FR 9515381
(43) Date de publication de la demande: 14.01.1998
(73) Titulaire: RHONE-POULENC RORER S.A., 92160 Antony (FR)
(72) Inventeur: Bouchard, Hervé, F-94200 Ivry-sur-Seine (FR); Bourzat, Jean-Dominique, F-94300 Vincennes (FR); Commerçon,Alain, F-94400 Vitry-sur-Seine (FR)
(74) Mandataire: Le Pennec, Magali
(86) Numéro de dépôt international: FR9600440
(87) Numéro de publication internationale: WO9630355

(56) Documents cités:
- EP-A- 0 639 577
- WO-A-94/18164

## Description

La présente invention concerne de nouveaux taxoïdes de formule générale : 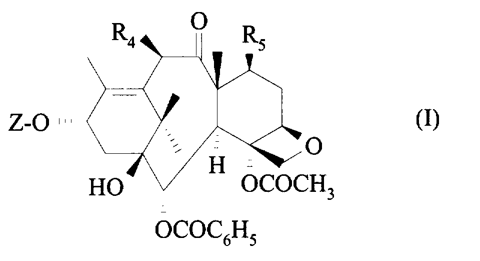 dans laquelle
Z représente un atome d'hydrogène ou un radical de formule générale : 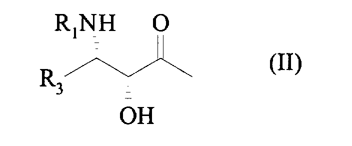 dans laquelle :
R₁ représente
   - un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle
   - ou un radical R₂-O-CO- dans lequel R₂ représente :
      - un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
      - un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
      - ou un radical hétérocyclyle saturé contenant 4 à 6 atomes de carbone éventuellement substitué par un ou plusieurs radicaux alcoyles contenant 1 à 4 atomes de carbone,
R₃ représente:
   - un radical alcoyle droit ou ramifié contenant 1 à 8 atomes de carbone,
   - alcényle droit ou ramifié contenant 2 à 8 atomes de carbone,
   - alcynyle droit ou ramifié contenant 2 à 8 atomes de carbone,
   - cycloalcoyle contenant 3 à 6 atomes de carbone,
   - phényle ou α- ou β-naphtyle
   éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, alcoylcarbamoyle, dialcoylcarbamoyle, cyano, nitro et trifluorométhyle,
   - ou un hétérocycle aromatique ayant 5 chaînons et contenant un ou plusieurs hétéroatomes, identiques ou différents, choisis parmi les atomes d'azote, d'oxygène ou de soufre et
   éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
R₄ représente :
   - un radical alcoxy contenant 1 à 6 atomes de carbone en chaîne droite ou ramifiée,
   - alcényloxy contenant 3 à 6 atomes de carbone en chaîne droite ou ramifiée,
   - alcynyloxy contenant 3 à 6 atomes de carbone en chaîne droite ou ramifiée,
   - cycloalcoyloxy contenant 3 à 6 atomes de carbone,
   - cycloalcényloxy contenant 4 à 6 atomes de carbone,
   ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone,
R₅ représente :
   - un radical alcoxy contenant 1 à 6 atomes de carbone en chaîne droite ou ramifiée (éventuellement substitué par un radical alcoxy contenant 1 à 4 atomes de carbone),
   - alcényloxy contenant 3 à 6 atomes de carbone,
   - alcynyloxy contenant 3 à 6 atomes de carbone,
   - cycloalcoyloxy contenant 3 à 6 atomes de carbone,
   - cycloalcényloxy contenant 3 à 6 atomes de carbone,
ces radicaux étant éventuellement substitués par un ou plusieurs atomes d'halogène ou par un radical alcoxy contenant 1 à 4 atomes de carbone, alcoylthio contenant 2 à 4 atomes de carbone, ou par un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone.

De préférence les radicaux aryles pouvant être représentés par R₃ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluoro-méthyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles.

De préférence les radicaux hétérocycliques pouvant être représentés par R₃ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

De préférence les radicaux R₄ et R₅, identiques ou différents, représentent des radicaux alcoxy droits ou ramifiés contenant 1 à 6 atomes de carbone éventuellement substitués par un radical méthoxy, éthoxy, éthylthio, carboxy, méthoxycarbonyle, éthoxycarbonyle, cyano, carbamoyle, N-méthyl-carbamoyle, N-éthylcarbamoyle, N,N-diméthylcarbamoyle, N,N-diéthylcarbamoyle, N-pyrrolidino-carbonyle ou N-pipéridinocarbonyle.

Plus particulièrement, la présente invention concerne les produits de formule générale (I) dans laquelle
Z représente un atome d'hydrogène ou un radical de formule générale (II) dans laquelle R₁ représente un radical benzoyle ou un radical R₂-O-CO- dans lequel R₂ représente un radical tert-butyle
et R₃ représente:
   - un radical alcoyle contenant 1 à 6 atomes de carbone,
   - alcényle contenant 2 à 6 atomes de carbone,
   - cycloalcoyle contenant 3 à 6 atomes de carbone,
   - phényle
   éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle
   - ou un radical furyle-2 ou -3,
   - thiényle-2 ou -3 ou
   - thiazolyle-2, -4 ou -5 et
R₄ et R₅, identiques ou différents, représentent chacun un radical alcoxy droit ou ramifié contenant 1 à 6 atomes de carbone.

Plus particulièrement encore, la présente invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ et R₅, identiques ou différents, représentent chacun un radical méthoxy, éthoxy ou propoxy.

Il était connu selon la demande de brevet WO 94/18164 un procédé de préparation de taxoïdes par condensation d'un motif β-lactam avec un noyau baccatine protégé en vue de préparer éventuellement le Taxotère.

Aucun moyen de préparer des noyaux taxoïdes porteurs de deux groupes alkoxy n'est décrit.

Il est également connu selon la demande de brevet EP 639577 une série de dérivés antitumoraux constitués de dérivés de la classe des taxanes porteurs en position 7,10 ou 2' d'un motif éther de phosphonooxyméthyl ou de méthylthiométhyl. Rien ne permet à la lecture de ce document d'envisager de remplacer 2 motifs spécifiques tels que décrits ci-dessus par 2 motifs alkoxy.

Les produits de formule générale (I) dans laquelle Z représente un radical de formule générale (II) présentent des propriétés antitumorales et antileucémiques remarquables.

Selon la présente invention, les nouveaux produits de formule générale (I) dans laquelle Z représente un radical de formule générale (II) peuvent être obtenus par estérification d'un produit de formule générale : 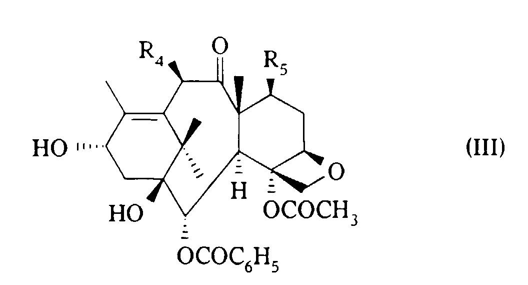 dans laquelle R₄ et R₅ sont définis comme précédemment, au moyen d'un acide de formule générale : 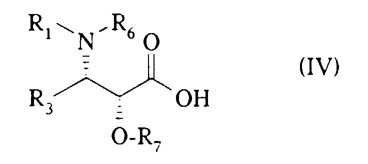 dans laquelle R₁ et R₃ sont définis comme précédemment, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, ou d'un dérivé de cet acide pour obtenir un ester de formule générale : 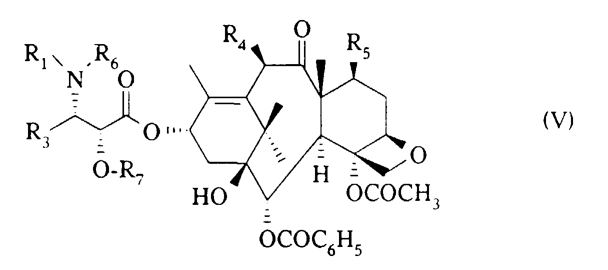 dans laquelle R₁, R₃, R₄, R₅, R₆ et R₇ sont définis comme précédemment, suivi du remplacement des groupements protecteurs représentés par R₇ et/ou R₆ et R₇ par des atomes d'hydrogène.

L'estérification au moyen d'un acide de formule générale (IV) peut être effectuée en présence d'un agent de condensation (carbodiimide, carbonate réactif) et d'un agent d'activation (aminopyridines) dans un solvant organique (éther, ester, cétones, nitriles, hydrocarbures aliphatiques, hydrocarbures aliphatiques halogénés, hydrocarbures aromatiques) à une température comprise entre -10 et 90°C.

L'estérification peut aussi être réalisée en utilisant l'acide de formule générale (IV) sous forme d'anhydride symétrique en opérant en présence d'un agent d'activation (aminopyridines) dans un solvant organique (éthers, esters, cétones, nitriles, hydrocarbures aliphatiques, hydrocarbures aliphatiques halogénés, hydrocarbures aromatiques) à une température comprise entre 0 et 90°C.

L'estérification peut aussi être réalisée en utilisant l'acide de formule générale (IV) sous forme d'halogénure ou sous forme d'anhydride mixte avec un acide aliphatique ou aromatique, éventuellement préparé in situ, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

De préférence, R₆ représente un atome d'hydrogène et R₇ représente un groupement protecteur de la fonction hydroxy ou bien R₆ et R₇ forment ensemble un hétérocycle saturé à 5 ou 6 chaînons.

Lorsque R₆ représente un atome d'hydrogène, R₇ représente de préférence un radical méthoxyméthyle, éthoxy-1 éthyle, benzyloxyméthyle, triméthylsilyle, triéthylsilyle, β-triméthylsilyléthoxyméthyle, benzyloxycarbonyle ou tétrahydropyrannyle.

Lorsque R₆ et R₇ forment ensemble un hétérocycle, celui-ci est de préférence un cycle oxazolidine éventuellement mono-substitué ou gem-disubstitué en position -2.

Le remplacement des groupements protecteurs R₇ et/ou R₆ et R₇ par des atomes d'hydrogène peut être effectué, selon leur nature de la manière suivante :
1) lorsque R₆ représente un atome d'hydrogène et R₇ représente un groupement protecteur de la fonction hydroxy, le remplacement des groupements protecteurs par des atomes d'hydrogène s'effectue au moyen d'un acide minéral (acide chlorhydrique, acide sulfurique, acide fluorhydrique) ou organique (acide acétique, acide méthanesulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C, ou au moyen d'une source d'ions fluorures tel qu'un complexe acide fluorhydrique-triéthylamine ou par hydrogénation catalytique,
2) lorsque R₆ et R₇ forment ensemble un hétérocycle saturé à 5 ou 6 chaînons et plus particulièrement un cycle oxazolidine de formule générale : 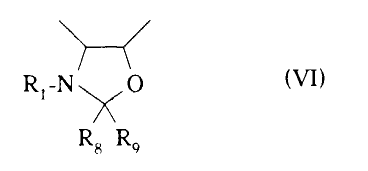 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, le remplacement du groupement protecteur formé par R₆ et R₇ par des atomes d'hydrogène peut être effectué, selon les significations de R₁, R₈ et R₉, de la manière suivante :
   a) lorsque R₁ représente un radical tert-butoxycarbonyle, R₈ et R₉, identiques ou différents, représentent un radical alcoyle ou un radical aralcoyle (benzyle) ou aryle (phényle), ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, le traitement de l'ester de formule générale (V) par un acide minéral ou organique éventuellement dans un solvant organique tel qu'un alcool conduit au produit de formule générale : 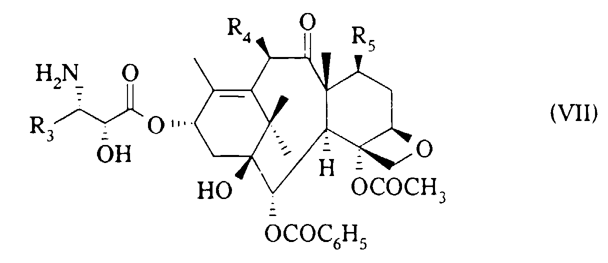 dans laquelle R₃, R₄ et R₅ sont définis comme précédemment, qui est acylé au moyen de chlorure de benzoyle dans lequel le noyau phényle est éventuellement substitué, de chlorure de thénoyle, de chlorure de furoyle ou d'un produit de formule générale :

      R₂-O-CO-X (VIII)

      dans laquelle R₂ est défini comme précédemment et X représente un atome d'halogène (fluor, chlore) ou un reste -O-R₂ ou -O-CO-O-R₂, pour obtenir un produit de formule générale (I) dans laquelle Z représente un radical de formule générale (II).
      De préférence, le produit de formule générale (V) est traité par l'acide formique à une température voisine de 20°C pour fournir le produit de formule générale (VII).
      De préférence, l'acylation du produit de formule générale (VII) au moyen d'un chlorure de benzoyle dans lequel le radical phényle est éventuellement substitué, de chlorure de thénoyle ou de chlorure de furoyle ou d'un produit de formule générale (VIII) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.
   b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, le remplacement du groupement protecteur formé par R₆ et R₇ par des atomes d'hydrogène s'effectue en présence d'un acide minéral (acide chlorhydrique, acide sulfurique) ou organique (acide acétique, acide méthanesulfonique, acide trifluorométhanesulfonique, acide p.toluènesulfonique) utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C.

Selon l'invention, les produits de formule générale (III), c'est-à-dire les produits de formule générale (I) dans laquelle Z représente un atome d'hydrogène, R₄ et R₅ sont définis comme précédemment, peuvent être obtenus à partir de la 10-désacétyl-baccatine III de formule :

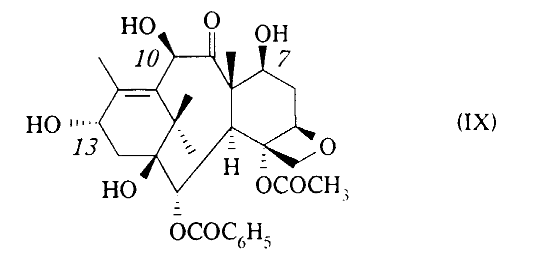

Il peut être particulièrement avantageux de protéger sélectivement les fonctions hydroxy en positions 7 et 13, par exemple sous forme d'un di-éther silylé qui peut être obtenu par action d'un halogénure de silyle de formule générale :

(R)₃-Si-Hal (X)

dans laquelle les symboles R, identiques ou différents, représentent un radical alcoyle contenant 1 à 6 atomes de carbone éventuellement substitué par un radical phényle, ou un radical cycloalcoyle contenant 3 à 6 atomes de carbone ou un radical phényle, sur la 10-désacétyl-baccatine III pour obtenir un produit de formule générale : 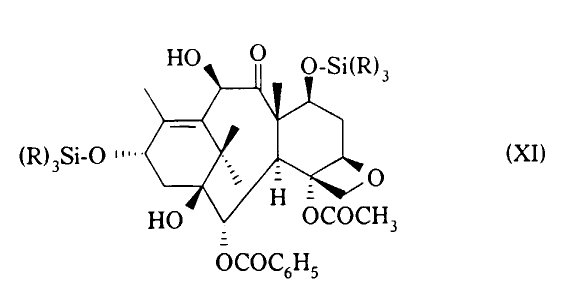 dans laquelle R est défini comme précédemment, puis action d'un produit de formule générale :

R'₄-X₁ (XII)

dans laquelle R'₄ représente un radical tel que R'₄-O est identique à R₄ défini comme précédemment et X₁ représente un reste d'ester réactif tel qu'un reste d'ester sulfurique ou sulfonique ou un atome d'halogène pour obtenir un produit de formule générale : 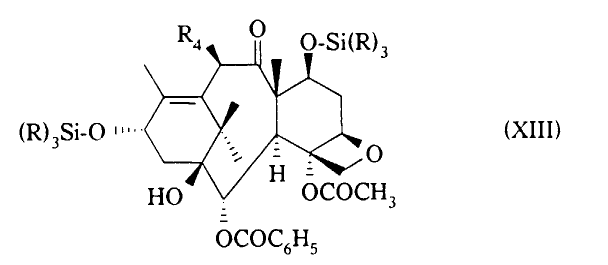 dans laquelle R et R₄ sont définis comme précédemment dont les groupements protecteurs silylés sont remplacés par des atomes d'hydrogène pour obtenir un produit de formule générale : 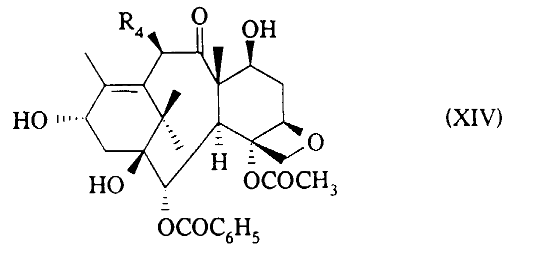 dans laquelle R₄ est défini comme précédemment, qui est éthérifié sélectivement en position 7 par action d'un produit de formule générale :

R'₅-X₂ (XV)

dans laquelle R'₅ représente un radical tel que R'₅-O est identique à R₅ défini comme précédemment et X₂ représente un atome d'halogène ou un reste d'ester réactif tel qu'un reste d'ester sulfurique ou sulfonique pour donner le produit de formule générale (III).

Généralement, l'action d'un dérivé silylé de formule générale (X) sur la 10-désacétyl-baccatine III est effectuée dans la pyridine ou la triéthylamine éventuellement en présence d'un solvant organique tel qu'un hydrocarbure aromatique comme le benzène, le toluène ou les xylènes à une température comprise entre 0°C et la température de reflux du mélange réactionnel.

Généralement, l'action d'un produit de formule générale (XII) sur un produit de formule générale (XI), est effectuée, après métallation de la fonction hydroxy en position 10 au moyen d'un hydrure de métal alcalin tel que l'hydrure de sodium, un amidure de métal alcalin tel que l'amidure de lithium ou d'un alcoylure de métal alcalin tel que le butyllithium, en opérant dans un solvant organique tel que le diméthylformamide ou le tétrahydrofurane à une température comprise entre 0 et 50°C.

Généralement le remplacement des groupements protecteurs silylés du produit de formule générale (XIII) par des atomes d'hydrogène s'effectue au moyen d'un acide tel que l'acide fluorhydrique ou l'acide trifluoroacétique en présence d'une base telle que la triéthylamine ou la pyridine éventuellement substituée par un ou plusieurs radicaux alcoyles contenant 1 à 4 atomes de carbone, éventuellement associée à un solvant organique inerte tel qu'un nitrile comme l'acétonitrile ou un hydrocarbure aliphatique halogéné comme le dichlorométhane à une température comprise entre 0 et 80°C.

Généralement l'action d'un produit de formule générale (XV) sur un produit de formule générale (XIV) s'effectue dans les conditions indiquées précédemment pour l'action d'un produit de formule générale (XII) sur un produit de formule générale (XI).

Selon l'invention, les produits de formule générale (I) dans laquelle Z représente un radical de formule générale (II), R₄ est défini comme précédemment et R₅ est défini comme précédemment, peuvent être obtenus à partir d'un produit de formule générale : 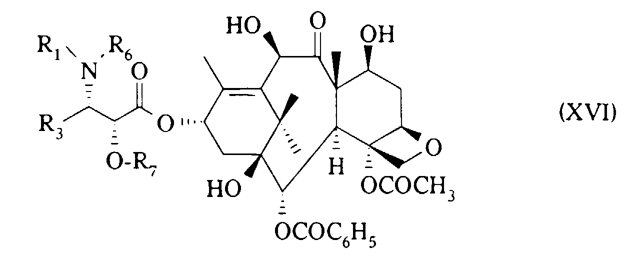 dans laquelle R₁, R₃, R₆ et R₇ sont définis comme précédemment par silylation en position 7 au moyen d'un produit de formule générale (X) pour obtenir un produit de formule générale : 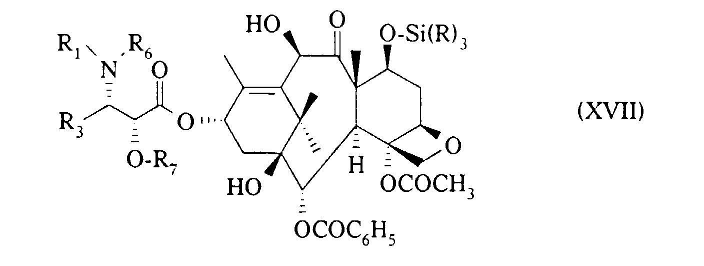 dans laquelle R, R₁, R₃, R₆ et R₇ sont définis comme précédemment, qui est fonctionnalisé en position 10 au moyen d'un produit de formule générale (XII) pour donner un produit de formule générale : 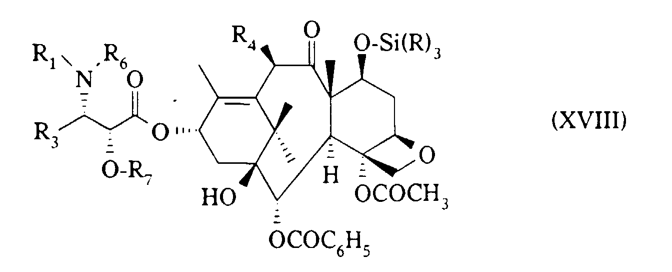 dans laquelle R, R₁, R₃, R₄, R₆ et R₇ sont définis comme précédemment dont le groupement protecteur silylé est remplacé par un atome d'hydrogène pour donner un produit de formule générale : 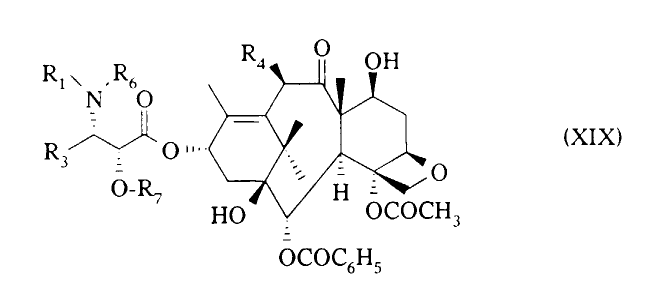 qui, par action d'un produit de formule générale (XV) conduit au produit de formule générale (V) dont les groupements protecteurs sont remplacés par des atomes d'hydrogène pour donner un produit de formule générale (I) dans laquelle Z représente un radical de formule générale (II).

Les réactions de silylation, de fonctionnalisation et de remplacement des groupements protecteurs par des atomes d'hydrogène sont effectuée dans des conditions analogues à celles décrites ci-dessus.

Les produits de formule générale (XVI) peuvent être obtenus dans les conditions décrites dans le brevet européen EP 0336 841 et les demandes internationales PCT WO 92/09589 et WO 94/07878 ou à partir des produits de formule générale : 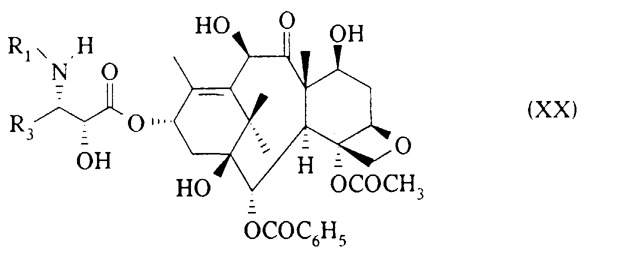 dans laquelle R₁ et R₃ sont définis comme précédemment selon les méthodes connues de protection de la fonction hydroxy de la chaîne latérale sans toucher au reste de la molécule.

Selon l'invention, les produits de formule générale (I) dans laquelle Z représente un atome d'hydrogène ou un radical de formule générale (II) peuvent être obtenus par action de nickel de Raney activé en présence d'un alcool aliphatique contenant 1 à 3 atomes de carbone ou d'un éther tel que le tétrahydrofurane ou le dioxane sur un produit de formule générale : 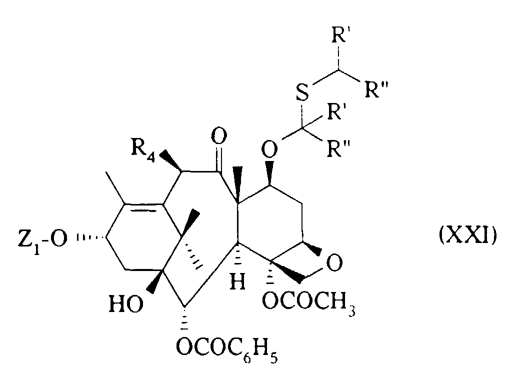 dans laquelle R4 est défini comme précédemment , R' et R", identiques ou différents, représentent un atome d'hydrogène ou un radical alcoyle contenant 1 à 6 atomes de carbone, alcényle contenant 2 à 6 atomes de carbone, alcynyle contenant 2 à 6 atomes de carbone, cycloalcoyle contenant 3 à 6 atomes de carbone ou cycloalcényle contenant 3 à 6 atomes de carbone éventuellement substitué, ou bien R' et R" forment ensemble avec l'atome de carbone auquel ils sont liés un radical cycloalcoyle contenant 3 à 6 atomes de carbone ou un radical cycloalcényle contenant 4 à 6 atomes de carbone, et Z₁ représente un atome d'hydrogène ou un radical de formule générale : 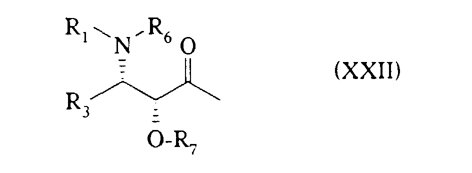 dans laquelle R₁, R₃, R₆ et R₇ sont définis comme précédemment et, pour obtenir un produit de formule générale : 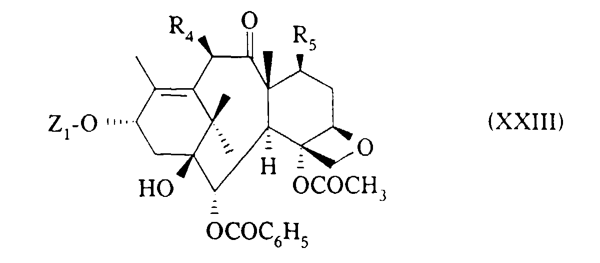 suivie, lorsque Z₁ représente un radical de formule générale (XXII), c'est-à-dire lorsque le produit de formule générale (XXIII) est identique au produit de formule générale (V), du remplacement des groupements protecteurs représentés par R₆ et/ou R₆ et R₇ par des atomes d'hydrogène dans les conditions décrites précédemment.

Généralement, l'action du nickel de Raney activé en présence d'un alcool aliphatique ou d'un éther est effectuée à une température comprise entre -10 et 60°C.

Selon l'invention, le produit de formule générale (XXI) dans laquelle Z₁ et R₄ sont définis comme précédemment peut être obtenu par action d'un sulfoxyde de formule générale : 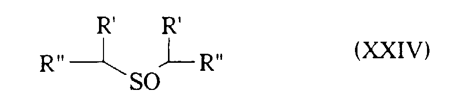 dans laquelle R' et R" sont définis comme précédemment, sur un produit de formule générale (XIX).

Généralement la réaction du sulfoxyde de formule générale (XXIV), de préférence le diméthylsulfoxyde, sur le produit de formule générale (XIX) s'effectue en présence d'un mélange d'acide acétique et d'anhydride acétique ou d'un dérivé de l'acide acétique tel qu'un acide halogénoacétique à une température comprise entre 0 et 50°C, de préférence voisine de 25°C.

Les produits de formule générale (I) obtenus par la mise en oeuvre du procédé selon l'invention peuvent être purifiés selon les méthodes connues telles que la cristallisation ou la chromatographie.

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) dans laquelle Z représente un radical de formule générale (II) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) dans laquelle Z représente un radical de formule générale (II) se sont montrés au moins aussi actifs que le taxol et le Taxotère.

In vivo, les produits de formule générale (I) dans laquelle Z représente un radical de formule générale (II) se sont montrés actifs chez la souris greffée par le mélanome B16 à des doses comprises entre 1 et 30 mg/kg par voie intrapéritonéale, ainsi que sur d'autres tumeurs liquides ou solides. Plus particulièrement, in vivo, sur le mélanome B16, le produit de formule générale (I) pour lequel R₁ représente un radical tert.butoxycarbonyle, R₃ représente un radical phényle et R₄ et R₅ représentent chacun un radical méthoxy présente un log cell kill de 2,9 alors que le produit de formule générale (I) pour lequel R₁ représente un radical tert.butoxycarbonyle, R₃ représente un radical phényle et R₄ représente un radical méthoxy et R₅ représente un radical hydroxy, décrit dans Tetrahedron Letters, 35, 5543 (1994) présente un log cell kill de 0,7.

Les nouveaux produits ont des propriétés anti-tumorales et plus particulièrement une activité sur les tumeurs qui sont résistantes au Taxol® ou au Taxotère®. De telles tumeurs comprennent les tumeurs du colon qui ont une expression élevée du gène mdr 1 (gène de la multi-drug resistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX, une lignée cellulaire sélectionnée pour sa résistance à la doxorubicine (DOX) qui exprime mdr 1.

Les exemples suivants illustrent la présente invention.

### EXEMPLE 1

A une suspension contenant 217,8 mg d'acétoxy-4α benzoyloxy-2α époxy-5β,20 dihydroxy-1β,13α diméthoxy-7β,10β oxo-9 taxène-11, 200 mg d'acide tert-butoxycarbonyl-3 (méthoxy-4 phényl)-2 phényl-4 oxazolidine-1,3 carboxylique-5 (2R,4S,5R) et 50 mg de tamis moléculaire 4Å en poudre dans 2 cm3 d'acétate d'éthyle, on ajoute successivement, à une température voisine de 20°C, 126 mg de dicyclohexylcarbodiimide, puis 14 mg de N,N'-diméthylamino-4 pyridine. La suspension obtenue est agitée à une température voisine de 20°C, sous atmosphère d'argon, pendant 16 heures puis concentrée à sec sous pression réduite (0,27 kPa) à une température voisine de 40°C. Le résidu obtenu est purifié par chromatographie à pression atmosphérique sur 50 g de silice (0,063-0,2 mm) contenus dans une colonne de 2 cm de diamètre (gradient d'élution : acétate d'éthyle-dichlorométhane de 10-90 à 40-60 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 271,8 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β diméthoxy-7β,10β oxo-9 taxène-11 yle-13α sous forme d'un solide blanc dont les caractéristiques sont les suivantes :
- spectre de R.M.N. ¹H (400 MHz ; CDCl₃ avec quelques gouttes de CD₃OD d4 : déplacements chimiques δ en ppm ; constantes de couplage J en Hz) : 1,02 (s, 9H : C(CH₃)₃) ; 1,10 (s, 3H : CH₃) ; 1,17 (s, 3H : CH₃) ; 1,63 (s, 3H : CH₃) ; de 1,65 à 1,85 et 2,60 (2 mts, 1H chacun : CH₂ en 6) ; 1,78 (mf, 3H : CH₃) ; 2,02 et 2,15 (2 dd, J = 14 et 9, 1H chacun : CH₂ en 14) ; 2,14 (s, 3H :CH₃) ; 3,22 et 3,35 (2 s, 3H chacun : OCH₃) ; 3,64 (d, J = 7, 1H : H en 3) ; 3,73 (mt, 1H : H en 7) ; 3,76 (s, 3H : ArOCH₃) ; 4,06 et 4,16 (2 d, J = 8,5, 1H chacun : CH₂ en 20) ; 4,53 (d, J = 5, 1H : H en 2') ; 4,67 (s, 1H : H en 10) ; 4,85 (d large, J = 10, 1H : H en 5) ; 5,36 (mt, 1H : H 3') ; 5,52 (d, J = 7, 1H : H en 2) ; 6,07 (mt, 1H : H en 13) ; 6,33 (mf, 1H : H en 5') ; 6,88 (d, J = 8, 2H : H aromatiques en ortho du OCH₃) ; de 7,25 à 7,40 (mt, 7H : H aromatiques en 3' et H aromatiques en méta du OCH₃) ; 7,43 (t, J = 7,5, 2H : OCOC₆H₅ H en méta) ; 7,58 (t, J = 7,5, 1H : OCOC₆H₅ H en para) ; 7,96 (d, J = 7,5, 2H : OCOC₆H₅ H en ortho).

Une solution de 446,3 mg de tert-butoxycarbonyl-3 (méthoxy-4 phényl)-2 phényl4 oxazolidine-1,3 carboxylate-5(2R,45,5R) d'acétoxy-4α benzoyloxy-2α époxy-5β,20 hydroxy-1β diméthoxy-7β,10β oxo-9 taxène-11 yle-13α dans 11,6 cm3 d'une solution 0,1N d'éthanol chlorhydrique est maintenue sous agitation à une température voisine de 0°C pendant 16 heures sous atmosphère d'argon. Le mélange réactionnel est alors dilué avec 40 cm3 de dichlorométhane et 5 cm3 d'eau distillée. Après décantation, la phase aqueuse est extraite avec 5cm3 de dichlorométhane. Les phases organiques sont rassemblées, séchées sur sulfate de magnésium, filtrées sur verre fritté puis concentrées à sec sous pression réduite (0,27 kPa) à une température voisine de 40°C. On obtient ainsi 424,2 mg d'un solide jaune pâle que l'on purifie par chromatographie préparative sur couche mince [12 plaques préparatives Merck, Kieselgel 60F254, épaisseur 1 mm, dépôt en solution dans un mélange méthanol-dichlorométhane (5-95 en volumes), en éluant par un mélange méthanol-dichlorométhane (5-95 en volumes)]. Après élution de la zone correspondant au produit principal par un mélange méthanol-dichlorométhane (15-85 en volumes), filtration sur verre fritté, puis évaporation des solvants sous pression réduite (0,27 kPa) à une température voisine de 40°C, on obtient 126 mg de tert-butoxycarbonylamino-3 hydroxy-2 phényl-3 propionate-(2R,3S) d'acétoxy-4α benzoyloxy-2α époxy-5β,20 hydroxy-1β diméthoxy-7β,10β oxo-9 taxène-11 yle-13α sous forme d'une meringue couleur ivoire dont les caractéristiques sont les suivantes :
- pouvoir rotatoire [α]^{D}₂₀ = -32,9 (c = 0,5 ; méthanol)
- spectre de R.M.N. ¹H (400 MHz ; CDCl₃ ; déplacements chimiques δ en ppm ; constantes de couplage J en Hz) : 1,23 (s, 3H : CH₃) ; 1,25 (s, 3H : CH₃) ; 1,39 (s, 9H : C(CH₃)₃) ; 1,70 (s, 1H : OH en 1) ; 1,75 (s, 3H : CH₃) ; 1,82 et 2,72 (2 mts, 1H chacun : CH₂ en 6) ; 1,91 (s, 3H : CH₃) ; 2,31 (AB limite, 2H : CH₂ en 14) ; 2,39 (s, 3H : COCH₃) ; 3,33 et 3,48 (2 s, 3H chacun : OCH₃) ; 3,48 (mt, 1H : OH en 2') ; 3,85 (d, J = 7, 1H : H 3) ; 3,88 (dd, J = 11 et 7, 1H : H 7) ; 4,20 et 4,33 (2 d, J = 8,5, 1H chacun : CH₂ en 20) ; 4,65 (mt, 1H : H en 2') ; 4,83 (s, 1H : H en 10 ); 5,00 (d large, J = 10, 1H : H en 5) ; 5,30 (d large, J = 10, 1H : H en 3') ; 5,47 (d, J = 10, 1H : CONH) ; 5,66 (d, J = 7, 1H : H en 2) ; 6,24 (t large, J = 9, 1H : H en 13) ; de 7,30 à 7,50 (mt, 5H : H aromatiques en 3') ; 7,52 (t, J = 7,5, 2H : OCOC₆H₅ H en méta) ; 7,63 (t, J = 7,5, 1H : OCOC₆H₅ H en para) ; 8,12 (d, J = 7,5, 2H : OCOC₆H₅ H en ortho).

L'acétoxy-4α benzoyloxy-2α époxy-5β,20 dihydroxy-1β,13α diméthoxy-7β,10β oxo-9 taxène-11 (ou 7β,10β-diméthoxy 10-désacétoxy-baccatine III) peut être préparé de la manière suivante :

A une solution de 500 mg d'acétoxy-4α benzoyloxy-2α époxy-5β,20 trihydroxy-1β,7β,13α méthoxy-10β oxo-9 taxène-11 dans 5 cm3 d'iodométhane et 0,5 cm3 de diméthylformamide, maintenue sous atmosphère d'argon, à une température voisine de 0°C, on ajoute par portions 86 mg d'hydrure de sodium à 50 % en poids dans l'huile de vaseline. Après 45 minutes à une température voisine de 0°C, le mélange réactionnel est dilué par 50 cm3 d'acétate d'éthyle et 8 cm3 d'eau distillée. Après décantation, la phase organique est lavée avec deux fois 8 cm3 d'eau distillée, puis 8 cm3 d'une solution aqueuse saturée en chlorure de sodium, séchée sur sulfate de magnésium, filtrée sur verre fritté, et concentrée à sec sous pression réduite (0,27 kPa) à une température voisine de 40°C. On obtient ainsi 570 mg d'un solide jaune pâle que l'on purifie par chromatographie à pression atmosphérique sur 50 g de silice (0,063-0,2 mm) contenus dans une colonne de 2,5 cm de diamètre en éluant avec un mélange méthanol-dichlorométhane (2-98 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 380 mg d'acétoxy-4α benzoyloxy-2α époxy-5β,20 dihydroxy-1β,13α diméthoxy-7β,10β oxo-9 taxène-11 sous forme d'un solide jaune pâle dont les caractéristiques sont les suivantes :
- spectre de R.M.N. ¹H (400 MHz ; CDCl₃ avec quelques gouttes de CD₃OD d4 : déplacements chimiques δ en ppm ; constantes de couplage J en Hz) : 1,03 (s, 3H : CH₃) ; 1,11 (s, 3H : CH₃) ; 1,65 (s, 3H : CH₃) ; 1,72 et 2,67 (2 mts, 1H chacun : CH₂ en 6) ; 2,05 (s, 3H : CH₃) ; 2,21 (AB limite, J = 14 et 9, 2H : CH₂ en 14) ; 2,25 (s, 3H : COCH₃) ; 3,26 et 3,40 (2 s, 3H chacun : OCH₃) ; 3,85 (d, J = 7, 1H : H en 3) ; 3,89 (dd, J = 11 et 6,5, 1H : H en 7) ; 4,12 et 4,25 (2 d, J = 8,5, 1H chacun : CH₂ en 20) ; 4,78 (t large, J = 9, 1H : H en 13) ; 4,83 (s, 1H : H en 10) ; 4,98 (d large, J = 10, 1H : H en 5) ; 5,53 (d, J = 7, 1H : H en 2) ; 7,43 (t, J = 7,5, 2H : OCOC₆H₅ H en méta) ; 7,56 (t, J = 7,5, 1H : OCOC₆H₅ H en para) ; 8,05 (d, J = 7,5, 2H : OCOC₆H₅ H en ortho).

L'acétoxy-4α benzoyloxy-2α époxy-5β,20 trihydroxy-1β,7β,13α méthoxy-10β oxo-9 taxène-11 (ou 10β-méthoxy 10-désacétoxy-baccatine III) peut être préparé de la manière suivante :

A une solution de 3,62 g d'acétoxy-4α benzoyloxy-2α époxy-5β,20 hydroxy-1β méthoxy-10β oxo-9 bistriéthylsilyloxy-7β,13α taxène-11 dans 30 cm3 de dichlorométhane, maintenue sous atmosphère d'argon, à une température voisine de 0°C, on ajoute lentement 50 cm3 de complexe fluorure d'hydrogène-triéthylamine (3HF.Et₃N). Après 48 heures à une température voisine de 20°C, le mélange réactionnel est versé sur une suspension de 100 cm3 d'une solution aqueuse sursaturée en hydrogénocarbonate de sodium maintenue à une température voisine de 0°C. Après décantation, la phase aqueuse est réextraite avec trois fois 80 cm3 de dichlorométhane, puis deux fois 80 cm3 d'acétate d'éthyle. Les phases organiques sont rassemblées, séchées sur sulfate de magnésium, filtrées sur sulfate de magnésium et concentrées à sec sous pression réduite (0,27 kPa) à une température voisine de 40°C. On obtient ainsi 3,45 g d'une meringue jaune que l'on purifie par chromatographie à pression atmosphérique sur 150 g de silice (0,063-0,2 mm) contenus dans une colonne de 3,5 cm de diamètre en éluant avec un mélange méthanol-dichlorométhane (5-95 en volumes) en recueillant des fractions de 35 cm3. Les fractions ne contenant que le produit cherché sont réunies et concentrées à sec sous pression réduite (0,27 kPa) à 40°C pendant 2 heures. On obtient ainsi 1,97 g d'acétoxy-4α benzoyloxy-2α époxy-5β,20 trihydroxy-1β,7β,13α méthoxy-10β oxo-9 taxène-11 sous forme d'un solide blanc dont les caractéristiques sont les suivantes :
- spectre de R.M.N. ¹H (400 MHz ; CDCl₃ ; déplacements chimiques δ en ppm ; constantes de couplage J en Hz) : 1,10 (s, 3H : CH₃) ; 1,19 (s, 3H : CH₃) ; 1,48 (d, J = 8,5, 1H : 0H en 13) ; 1,70 (s, 3H : CH₃) ; 1,81 et 2,61 (2 mts, 1H chacun : CH₂ en 6) ; 2,09 (d, J = 5, 1H : OH en 7) ; 2,11 (s, 3H : CH₃) ; 2,30 (s, 3H : COCH₃) ; 2,32 (d, J = 9, 2H : CH₂ en 14) ; 3,48 (s, 3H : OCH₃) ; 3,97 (d, J = 7, 1H : H en 3); 4,18 et 4,33 (2 d, J = 8,5, 1H chacun : CH₂ en 20) ; 4,31 (mt, 1H : H en 7) ; 4,93 (mt, 1H : H en 13); 4,99 (s, 1H : H en 10) ; 5.01 (d large, J = 10, 1H : H en 5) ; 5,66 (d, J = 7, 1H : H en 2) ; 7,49 (t, J = 7,5, 2H : OCOC₆H₅ H en méta) ; 7,63 (t, J = 7,5, 1H : OCOC₆H₅ Hen para) ; 8,12 (d, J = 7,5, 2H : OCOC₆H₅ H en ortho).

L'acétoxy-4α benzoyloxy-2α époxy-5β,20 hydroxy-1β méthoxy-10β oxo-9 bistriéthylsilyloxy-7β,13α taxène-11 (ou 10β-méthoxy 10-désacétoxy 7,13-bistriéthylsilyl-baccatine III) peut être préparé de la manière suivante :

A une solution de 5 g d'acétoxy-4α benzoyloxy-2α époxy-5β,20 dihydroxy-1β,10β oxo-9 bistriéthylsilyloxy-7β,13α taxène-11 dans 25 cm3 d'iodométhane, maintenue sous atmosphère d'argon, à une température voisine de 0°C, on ajoute par portions 375 mg d'hydrure de sodium à 50 % en poids dans l'huile de vaseline. La solution est maintenue sous agitation pendant 45 minutes à une température voisine de 0°C, puis pendant 5 heures 30 minutes à une température voisine de 20°C. Le mélange réactionnel est de nouveau refroidi à une température voisine de à 0°C, et l'on ajoute par portions 125 mg d'hydrure de sodium à 50 % en poids dans l'huile de vaseline. Après 1 heure à 20°C, puis 18 heures à 5°C, le mélange réactionnel est dilué par addition de 50 cm3 de dichlorométhane, versé sur 50 cm3 d'une solution aqueuse saturée en chlorure d'ammonium et décanté. La phase aqueuse est extraite par 2 fois 30 cm3 de dichlorométhane, puis les phases organiques sont rassemblées, lavées avec 10 cm3 d'eau distillée, séchées sur sulfate de magnésium, filtrées sur verre fritté, et concentrées à sec sous pression réduite (0,27 kPa) à une température voisine de 40°C. On obtient ainsi 5,15 g d'une meringue jaune que l'on purifie par chromatographie à pression atmosphérique sur 300 g de silice (0,063-0,2 mm) contenus dans une colonne de 5 cm de diamètre (gradient d'élution : acétate d'éthyle-dichlorométhane de 0-100 à 10-90 en volumes) en recueillant des fractions de 30 cm3. Les fractions ne contenant que le produit cherché sont réunies et concentrées à sec sous pression réduite (0,27kPa) à 40°C pendant 2 heures. On obtient ainsi 3,62 g d'acétoxy-4α benzoyloxy-2α époxy-5β,20 hydroxy-1β méthoxy-10β oxo-9 bistriéthylsilyloxy7β,13α taxène-11 sous forme d'une meringue jaune pâle dont les caractéristiques sont les suivantes :
- spectre de R.M.N. ¹H (600 MHz ; CDCl₃ ; déplacements chimiques δ en ppm ; constantes de couplage J en Hz) : 0,58 et 0,69 (2 mts, 6H chacun : CH₂ éthyle) ; 0,97 et 1,04 (2 t, J = 7,5, 9H chacun : CH₃ éthyle) ; 1,15 (s, 3H : CH₃) ; 1,18 (s, 3H : CH₃) ; 1,58 (s, 1H : 0H en 1) ; 1,68 (s, 3H : CH₃) ; 1,89 et 2,48 (2 mts, 1H chacun : CH₂ en 6) ; 2,04 (s, 3H : CH₃) ; 2,15 et 2,23 (2 dd, J = 16 et 9, 1H chacun : CH₂ en 14) ; 2,29 (s, 3H : COCH₃) ; 3,40 (s, 3H : OCH₃) ; 3,83 (d, J = 7, 1H : H en 3) ; 4,15 et 4,30 (2 d, J = 8,5, 1H chacun : CH₂ en 20) ; 4,43 (dd, J = 11 et 7, 1H : H en 7) ; 4,91 (s, 1H : H en 10) ; 4,96 (d large, J = 10, 1H : H en 5) ; 5,01 (t large, J = 9, 1H : H en 13) ; 5,62 (d, J = 7, 1H : H en 2) ; 7,46 (t, J = 7,5, 2H : OCOC₆H₅ H en méta) ; 7,60 (t, J = 7,5, 1H : OCOC₆H₅ H en para) ; 8,09 (d, J = 7,5, 2H : OCOC₆H₅ H en ortho).

L'acétoxy-4α benzoyloxy-2α époxy-5β,20 dihydroxy-1β,10β oxo-9 bistriéthylsilyloxy-7β,13α taxène-11 (ou 10-désacétyl 7,13-bistriéthylsilyl-baccatine (III) peut être préparé de la manière suivante :

A une solution de 14 g d'acétoxy-4α benzoyloxy-2α époxy-5β,20 tétrahydroxy-1β,7β,10β,13α oxo-9 taxène-11 (10-désacétyl-baccatine III) dans 50 cm3 de pyridine anhydre, maintenue sous atmosphère d'argon, à une température voisine de 20°C, on ajoute 10,8 cm3 de chlorure de triéthylsilyle. Après 17 heures à une température voisine de 20°C, le mélange réactionnel est porté à une température voisine de 115°C, puis on ajoute 10,8 cm3 de chlorure de triéthylsilyle. Après 3 heures 15 minutes à une température voisine de 115°C, le mélange réactionnel est ramené jusqu'à une température voisine de 20°C, dilué avec 30 cm3 d'acétate d'éthyle et 100 cm3 d'eau distillée. Après décantation, la phase aqueuse est extraite avec 2 fois 50 cm3 d'acétate d'éthyle. Les phases organiques sont rassemblées, lavées avec 50 cm3 d'une solution aqueuse saturée en chlorure de sodium, séchées sur sulfate de magnésium, filtrées sur verre fritté puis concentrées à sec sous pression réduite (0,27 kPa) à une température voisine de 40°C. On obtient ainsi 63,1 g d'une huile brune que l'on purifie par chromatographie à pression atmosphérique sur 800 g de silice (0,063-0,2 mm) contenus dans une colonne de 7 cm de diamètre (gradient d'élution : acétate d'éthyle-dichlorométhane de 0-100 à 5-95 en volumes) en recueillant des fractions de 60 cm3. Les fractions ne contenant que le produit cherché sont réunies et concentrées à sec sous pression réduite (0,27 kPa) à 40°C pendant 2 heures. On obtient ainsi 9,77 g d'acétoxy-4α benzoyloxy-2α époxy-5β,20 dihydroxy-1β,10β oxo-9 bistriéthylsilyloxy-7β,13α taxène-11 sous forme d'une meringue crème dont les caractéristiques sont les suivantes :
- spectre de R.M.N. ¹H (400 MHz ; CDCl₃ ; déplacements chimiques δ en ppm ; constantes de couplage J en Hz) : 0,55 et 0,68 (2 mts, 6H chacun : CH₂ éthyle) ; 0,94 et 1,03 (2 t, J = 7,5, 9H chacun : CH₃ éthyle) ; 1,08 (s, 3H : CH₃) ; 1,17 (s, 3H : CH₃) ; 1,58 (s, 1H : OH en 1) ; 1,73 (s, 3H : CH₃) ; 1,91 et 2,57 (2 mts, 1H chacun : CH₂ en 6) ; 2,04 (s, 3H : CH₃) ; 2,12 et 2,23 (2 dd, J = 16 et 9, 1H chacun : CH₂ en 14) ; 2,30 (s, 3H : COCH₃) ; 3,88 (d, J = 7, 1H : H en 3) ; 4,16 et 4,32 (2 d, J = 8,5, 1H chacun : CH₂ en 20) ; 4,27 (d, J = 1, 1H : OH en 10); 4,40 (dd, J = 11 et 7, 1H : H en 7) ; 4,95 (d large, J = 10, 1H : H en 5) ; 4,95 (mt, 1H : H en 13) ; 5,16 (d, J = 1, 1H : H en 10); 5,60 (d, J = 7, 1H : H en 2) ; 7,46 (t, J = 7,5, 2H : OCOC₆H₅ H en méta) ; 7,60 (t, J = 7,5, 1H : OCOC₆H₅ H en para) ; 8,09 (d, J = 7,5, 2H : OCOC₆H₅ H en ortho).

### EXEMPLE 2

340 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β diméthoxy-7β,10β oxo-9 taxène-11 yle-13α sont dissous dans 8 cm3 d'une solution éthanolique 0,1N d'acide chlorhydrique à 1 % d'eau. La solution ainsi obtenue est agitée pendant 13 heures à une température voisine de 20°C puis pendant 80 heures à 4°C et additionnée de 20 cm3 de dichlorométhane. La phase organique est séparée par décantation et lavée successivement par 3 fois 5 cm3 d'une solution aqueuse saturée d'hydrogénocarbonate de sodium, séchée sur sulfate de magnésium, filtrée et concentrée à sec sous pression réduite (2,7 kPa) à 40°C. On obtient 300 mg d'une meringue blanche que l'on purifie par chromatographie sur gel de silice déposé sur plaques [(gel de 1mm d'épaisseur, plaques de 20 x 20 cm, éluant : dichlorométhane-méthanol (95-5 en volumes)] par fractions de 80 mg (4 plaques). Après localisation aux rayons U.V. de la zone correspondant au produit cherché adsorbé, cette zone est grattée et la silice recueillie est lavée sur verre fritté par 10 fois 5 cm3 d'acétate d'éthyle. Les filtrats sont réunis et concentrés à sec sous pression réduite (2,7 kPa) à 40°C. On obtient une meringue blanche que l'on repurifie selon la même technique [(3 plaques : 20 x 20 x I mm; éluant : dichlorométhane-acétate d'éthyle (90-10 en volumes)]. On obtient ainsi 205 mg de tert-butoxycarbonylamino-3 hydroxy-2 phényl-3 propionate-(2R,3S) d'acétoxy-4α benzoyloxy-2α époxy-5β,20 hydroxy-1β diméthoxy-7β,10β oxo-9 taxène-11 yle-13α sous forme d'une meringue blanche dont les caractéristiques sont les suivantes :
- pouvoir rotatoire : [α]^{D}₂₀ = - 33 (c = 0,5 ; méthanol).
- spectre de R.M.N. : ¹H (400 MHz ; CDCl₃ ; déplacements chimiques δ en ppm ; constantes de couplage J en Hz) . 1,23 (s, 3H : -CH₃) ; 1,25 (s, 3H : -CH₃) ; 1,39 [s, 9H : -C(CH₃)₃] ; 1,70 (s, 1H : -OH en 1) ; 1,75 (s, 3H : -CH₃) ; 1,82 et 2,72 (2 mts, 1H chacun : -CH₂ en 6) ; 1,91 (s, 3H : -CH₃) ; 2,31 (AB limite, 2H : -CH₂ en 14) ; 2,39 (s, 3H : -COCH₃) ; 3,33 et 3,48 (2 s, 3H chacun : -OCH₃) ; 3,48 (mt, 1H : OH 2') ; 3,85 (d, J = 7, 1H : -H en 3) ; 3,88 (dd, J = 11 et 7, 1H : -H en 7) ; 4,20 et 4,33 (2 d, J = 8,5, 1H chacun : -CH₂ en 20) ; 4,65 (mt, 1H : -H en 2') ; 4,83 (s, 1H : -H en 10) ; 5,00 (d large, J = 10, 1H : -H en 5) ; 5,30 (d large, J = 10, 1H : -H en 3') ; 5,47 (d, J = 10, 1H : -CONH-) ; 5,66 (d, J = 7, 1H : -H en 2) ; 6,24 (t large, J = 9, 1H : -H en 13) ; de 7,30 à 7,50 (mt, 5H : -C₆H₅ en 3') ; 7,52 [t, J = 7,5, 2H : -OCOC₆H₅(-H en 3 et H en 5)] ; 7,63 [t, J = 7,5, 1H : -OCOC₆H₅(-H en 4)] ; 8,12 [d, J = 7,5, 2H :-OCOC₆H₅(-H en 2 et H en 6)].

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β diméthoxy-7β,10β oxo-9 taxène-11 yle-13α peut être préparé de la manière suivante :

A une solution de 1 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β bis(méthylthiométhoxy)-7β,10β oxo-9 taxène-11 yle-13α dans 100 cm3 d'éthanol anhydre, maintenue sous atmosphère d'argon et sous agitation, on ajoute, à une température voisine de 20°C, 100 cm3 d'une suspension éthanolique de nickel activé selon Raney (obtenue à partir de 80 cm3 de la suspension aqueuse commerciale à environ 50 % par lavage successifs, jusqu'à un pH voisin de 7, par 15 fois 100 cm3 d'eau distillée et par 5 fois 100 cm3 d'éthanol). Le milieu réactionnel est maintenu sous agitation pendant 24 heures à une température voisine de 20°C puis filtré sur verre fritté. Le verre fritté est lavé par 4 fois 80 cm3 d'éthanol, les filtrats sont réunis et concentrés à sec sous pression réduite (2,7 kPa) à 40°C. On obtient 710 mg d'une meringue jaune que l'on purifie par chromatographie sur 60 g de silice (0,063-0,2 mm) contenus dans une colonne de 2,5 cm de diamètre [éluant : dichlorométhane-acétate d'éthyle (90-10 en volumes)] en recueillant des fractions de 6 cm3. Les fractions ne contenant que le produit cherché sont réunies et concentrées à sec sous pression réduite (2,7 kPa) à 40°C. On obtient ainsi 350 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β diméthoxy-7β,10β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β bis(méthylthiométhoxy)-7β,10β oxo-9 taxéne-11 yle-13α peut être préparé de la manière suivante :

A une solution de 3,1 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 trihydroxy-1β,7β,10β oxo-9 taxène-11 yle-13α en solution dans 102 cm3 de diméthylsulfoxyde, maintenue sous atmosphère d'argon et sous agitation, on ajoute, à une température voisine de 20°C, 2,3 cm3 d'acide acétique et 7,55 cm3 d'anhydride acétique. Le mélange réactionnel est maintenu sous agitation pendant 7 jours à une température voisine de 20°C puis versé dans un mélange de 500 cm3 d'eau distillée et de 250 cm3 de dichlorométhane. On additionne ensuite sous bonne agitation 30 cm3 d'une solution aqueuse saturée de carbonate de potassium jusqu'à un pH voisin de 7. Après 10 minutes d'agitation, la phase organique est séparée par décantation et on réextrait la phase aqueuse par 2 fois 250 cm3 de dichlorométhane. Les phases organiques sont réunies, lavées par 250 cm3 d'eau distillée, séchées sur sulfate de magnésium, filtrées et concentrées à sec sous pression réduite (2,7 kPa) à 40°C. On obtient 5,2 g d'une huile jaune pâle que l'on purifie par chromatographie sur 200 g de silice (0,063-0,4 mm) contenus dans une colonne de 3 cm de diamètre [éluant : dichlorométhane-méthanol (99-1 en volumes)] en recueillant des fractions de 50 cm3. Les fractions ne contenant que le produit cherché sont réunies et concentrées à sec sous pression réduite (2,7 kPa) à 40°C. On obtient ainsi 1,25 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β bis(méthylthiométhoxy)-7β,10β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 trihydroxy-1β,7β,10β oxo-9 taxène-11 yle-13α peut être préparé de la manière suivante :

Une solution de 5,1 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β oxo-9 bis-(trichloro-2,2,2 éthoxy)carbonyloxy-7β,10β taxène-11 yle-13α dans un mélange de 100 cm3 de méthanol et de 100 cm3 d'acide acétique est chauffée, sous agitation et sous atmosphère d'argon, jusqu'à une température voisine de 60°C, puis additionnée de 10 g de zinc en poudre. Le mélange réactionnel est ensuite agité pendant 15 minutes à 60°C puis refroidi à une température voisine de 20°C et filtré sur verre fritté garni de célite. Le verre fritté est lavé par 2 fois 15 cm3 de méthanol. Le filtrat est concentré à sec sous pression réduite (2,7 kPa) à une température voisine de 40°C. Le résidu est additionné de 50 cm3 d'acétate d'éthyle et de 25 cm3 d'une solution aqueuse saturée d'hydrogénocarbonate de sodium. La phase organique est séparée par décantation et lavée successivement par 25 cm3 d'une solution aqueuse saturée d'hydrogénocarbonate de sodium et par 25 cm3 d'eau distillée puis séchée sur sulfate de magnésium, filtrée sur verre fritté et concentrée à sec sous pression réduite (2,7 kPa) à 40°C. On obtient ainsi 3,1 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 trihydroxy-1β,7β,10β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R)) d'acétoxy-4α benzoyloxy-2α époxy-5β,20 hydroxy-1β oxo-9 bis-(trichloro-2,2,2 éthoxy)carbonyloxy-7β,10β taxène-11 yle-13α peut être préparé dans les conditions décrites dans le brevet WO 94/07878.

### EXEMPLE 3

A une suspension contenant 135 mg d'acétoxy-4α benzoyloxy-2α époxy-5β,20 éthoxy-10β dihydroxy-1β,13α méthoxy-7β oxo-9 taxène-11, 120 mg d'acide tert-butoxycarbonyl-3 (méthoxy-4 phényl)-2 phényl-4 oxazolidine-1,3 carboxylique-5-(2R,4S,5R) et 50 mg de tamis moléculaire 4Å en poudre dans 1 cm3 de toluène anhydre, on ajoute successivement, à une température voisine de 20°C, 76 mg de dicyclohexylcarbodiimide, puis 8,5 mg de N,N'-diméthylamino-4 pyridine. La suspension obtenue est agitée à une température voisine de 20°C sous atmosphère d'argon pendant 1 heure puis purifiée par dépôt direct sur une colonne de chromatographie à pression atmosphérique sur 30 g de silice (0,063-0,2 mm) contenus dans une colonne de 2,5 cm de diamètre (gradient d'élution : acétate d'éthyle-dichlorométhane de 2-98 à 10-90 en volumes) en recueillant des fractions de 10 cm3. Les fractions ne contenant que le produit cherché sont réunies et concentrées à sec sous pression réduite (2,7 kPa) à 40°C pendant 2 heures. On obtient ainsi 320,6 mg d'un solide blanc qui est purifié par chromatographie préparative sur couche mince : 10 plaques préparatives Merck, Kieselgel 60F254, épaisseur 0,5 mm, dépôt en solution dans le dichlorométhane, en éluant par un mélange méthanol-dichlorométhane (3-97 en volumes). Après élution des zones correspondants aux produits principaux par un mélange méthanol-dichlorométhane (15-85 en volumes), filtration sur coton, puis évaporation des solvants sous pression réduite (2,7 kPa) à une température voisine de 40°C, on obtient 47,7 mg d'acétoxy-4α benzoyloxy-2α époxy-5β,20 éthoxy-10β dihydroxy-1β,13α méthoxy-7β oxo-9 taxène-11 sous forme d'un solide crème et 37 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 éthoxy-10β hydroxy-1β méthoxy-7β oxo-9 taxène-11 yle-13α sous forme d'une meringue blanche dont les caractéristiques sont les suivantes :
- spectre de R.M.N. ¹H (600 MHz ; CDCl₃ ; à une température de 333°K ; déplacements chimiques δ en ppm ; constantes de couplage J en Hz) : 1,09 (s, 9H : C(CH₃)₃) ; 1,19 (s, 3H : CH₃) ; 1,21 (s, 3H : CH₃) ; 1,27 (t, J = 7, 3H : CH₃ de l'éthyle) ; 1,43 (s, 1H : OH en 1) ; 1,62 (s, 3H : CH₃) ; 1,68 (s, 3H : CH₃) ; 1,77 et 2,63 (2 mts, 1H chacun : CH₂ en 6) ; 1,86 (s, 3H : COCH₃) ; 2,13 et 2,22 (2 dd, J = 16 et 9, 1H chacun : CH₂ en 14) ; 3,27 (s, 3H : OCH₃) ; 3,45 et 3,68 (2 mts, 1H chacun : CH₂ de l'éthyle) ; 3,76 (d, J = 7, 1H : H 3) ; 3,81 (s, 3H : ArOCH₃) ; 3,85 (dd, J = 11 et 7, 1H : H en 7) ; 4,13 et 4,23 (2 d, J = 8,5, 1H chacun : CH₂ en 20); 4,58 (d, J = 4,5, 1H : H en 2') ; 4,83 (s, 1H : H en 10); 4,90 (d large, J = 10, 1H : H en 5) ; 5,46 (d, J = 4,5, 1H : H en 3') ; 5,60 (d, J = 7 Hz, 1H : H 2) ; 6,13 (t large, J = 9 Hz, 1H : H 13) ; 6,38 (s, 1H : H 5') ; 6,92 (d, J = 8,5, 2H : H aromatiques en ortho du OCH₃) ; de 7,30 à 7,50 (mt, 9H : H aromatiques en 3' - H aromatiques en méta du OCH₃ et OCOC₆H₅ H en méta) ; 7,59 (t, J = 7,5, 1H : OCOC₆H₅ H en para) ; 8,03 (d, J = 7,5, 2H : OCOC₆H₅ H en ortho).

Une solution de 48 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5(3,20 éthoxy-10β hydroxy-1β méthoxy-7β oxo-9 taxène-11 yle-13α dans 0,5 cm3 d'acétate d'éthyle et 0,004 cm3 d'acide chlorhydrique concentré à 37 % est maintenue sous agitation à une température voisine de 20°C pendant 1,5 heures sous atmosphère d'argon. Le mélange réactionnel est alors purifié par chromatographie préparative sur couche mince : dépôt du mélange réactionnel brut sur 5 plaques préparatives Merck, Kieselgel 60F254, épaisseur 0,5 mm, en éluant par un mélange méthanol-dichlorométhane (4-96 en volumes). Après élution de la zone correspondant au produit principal par un mélange méthanol-dichlorométhane (15-85 en volumes), filtration sur coton, puis évaporation des solvants sous pression réduite (2,7 kPa) à une température voisine de 40°C, on obtient 28,5 mg de tert-butoxycarbonylamino-3 hydroxy-2 phényl-3 propionate-(2R,3S) d'acétoxy-4α benzoyloxy-2α époxy-5β,20 éthoxy-10β hydroxy-1β méthoxy-7β oxo-9 taxène-11 yle-13α sous forme d'une meringue couleur ivoire dont les caractéristiques sont les suivantes :
- spectre de R.M.N.¹H: (400 MHz ; CDCl₃ ; déplacements chimiques δ en ppm ; constantes de couplage J en Hz): 1,22 (s, 3H : CH₃) ; 1,25 (s, 3H : CH₃) ; 1,32 (t, J = 7, 3H : CH₃ de l'éthyle) ; 1,38 (s, 9H : C(CH₃)₃) ; 1,64 (s, 1H : OH en 1) ; 1,73 (s, 3H : CH₃) ; 1,80 et 2,70 (2 mts, 1H chacun : CH₂ en 6) ; 1,88 (s, 3H : CH₃) ; 2,30 (mt, 2H : CH₂ en 14) ; 2,38 (s, 3H : COCH₃) ; 3,31 (s, 3H : OCH₃) ; 3,44 (mf, 1H : OH en 2') ; 3,50 et 3,70 (2 mts, 1H chacun : OCH₂ de l'éthyle); 3,84 (d, J = 7,5, 1H : H en 3) ; 3,87 (dd, J = 11 et 6,5, 1H : H en 7) ; 4,18 et 4,32 (2 d, J = 8,5, 1H chacun : CH₂ en 20) ; 4,64 (mt, 1H : H en 2') ; 4,90 (s, 1H : H en 10) ; 4,98 (d large, J = 10, 1H : H en 5) ; 5,28 (d large, J = 10, 1H : H en 3') ; 5,42 (d, J = 10, 1H : CONH) ; 5,64 (d, J = 7,5, 1H : H en 2) ; 6,22 (t large, J = 9, 1H : H en 13) ; de 7,25 à 7,45 (mt, 5H : H aromatiques en 3') ; 7,50 (t, J = 7,5, 2H : OCOC₆H₅ H en méta) ; 7,62 (t, J = 7,5, 1H : OCOC₆H₅ H en para) ; 8,12 (d, J = 7,5, 2H : OCOC₆H₅ H en ortho).

L'acétoxy-4α benzoyloxy-2α époxy-5β,20 éthoxy-10β dihydroxy-1β,13α méthoxy-7β oxo-9 taxène-11 (ou éthoxy-10β méthoxy-7β désacétoxy-10 baccatine III) peut être préparé de la manière suivante :

A une solution de 235 mg d'acétoxy-4α benzoyloxy-2α époxy-5β,20 trihydroxy-1β,7β,13α éthoxy-10β oxo-9 taxène-11 dans 2,5 cm3 d'iodométhane et 1 cm3 de diméthylformamide, maintenue sous atmosphère d'argon, à une température voisine de 0°C, on ajoute par portions 43 mg d'hydrure de sodium à 50 % en poids dans l'huile de vaseline. Après 30 minutes à une température voisine de 0°C, le mélange réactionnel est dilué par 40 cm3 d'acétate d'éthyle, 6 cm3 d'eau distillée et 8 cm3 d'une solution aqueuse saturée en chlorure d'ammonium. Après décantation, la phase organique est lavée avec trois fois 8 cm3 d'eau distillée, puis 8 cm3 d'une solution aqueuse saturée en NaCl, séchée sur sulfate de magnésium, filtrée sur verre fritté, et concentrée à sec sous pression réduite (2,7 kPa) à une température voisine de 40°C. On obtient ainsi 268 mg d'un solide jaune que l'on purifie par chromatographie à pression atmosphérique sur 30 g de silice (0,063-0,2 mm) contenus dans une colonne de 2,5 cm de diamètre (gradient d'élution : acétate d'éthyle-dichlorométhane de 0-100 à 15-85 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 380 mg d'acétoxy-4α benzoyloxy-2α époxy-5β,20 éthoxy-10β dihydroxy-1β,13α méthoxy-7β oxo-9 taxène-11 sous forme d'une poudre blanche dont les caractéristiques sont les suivantes :
- spectre de R.M.N. ¹H (300 MHz ; CDCl₃ avec ajout de quelques gouttes de CD₃OD d4 ; déplacements chimiques δ en ppm ; constantes de couplage J en Hz) : 0,99 (s, 3H : CH₃) ; 1,09 (s, 3H : CH₃) ; 1,22 (t, J = 7, 3H : CH₃ de l'éthyle) ; 1,62 (s, 3H : CH₃) ; 1,68 et 2,66 (2 mts, 1H chacun : CH₂ 6) ; 2,03 (s, 3H : CH₃) ; 2,13 et 2,22 (2 dd, J =16 et 9, 1H chacun : CH₂ en 14) ; 2,23 (s, 3H: COCH₃) ; 3,23 (s, 3H : OCH₃) ; de 3,40 à 3,65 (mt, 2H : CH₂ de l'éthyle) ; 3,84 (d, J = 7,5, 1H : H en 3) ; 3,88 (dd, J = 10 et 6,5, 1H : H en 7) ; 4,10 et 4,23 (2 d, J = 8,5, 1H chacun : CH₂ 20) ; 4,75 (t large, J = 9, 1H : H en 13) ; 4,90 (s, 1H : H en 10) ; 4,97 (d large, J = 10, 1H : H en 5) ; 5,51 (d, J = 7,5, 1H : H en 2) ; 7,42 (t, J = 7,5, 2H : OCOC₆H₅ H en méta) ; 7,53 (t, J = 7,5, 1H : OCOC₆H₅ H en para) ; 8,03 (d, J = 7,5, 2H : OCOC₆H₅ H en ortho).

L'acétoxy-4α benzoyloxy-2α époxy-5β,20 trihydroxy-1β,7β,13α éthoxy-10β oxo-9 taxène-11 (ou éthoxy-10β désacétoxy-10 baccatine III) peut être préparé de la manière suivante :

A une solution de 591 mg d'acétoxy-4α benzoyloxy-2α époxy-5β,20 hydroxy-1β éthoxy-10β oxo-9 bistriéthylsilyloxy-7β,13α taxène-11 dans 6 cm3 de dichlorométhane, maintenue sous atmosphère d'argon, à une température voisine de 20°C, on ajoute 9 cm3 de complexe fluorure d'hydrogène-triéthylamine (3HF.Et₃N). Après 21 heures à une température voisine de 20°C, le mélange réactionnel est dilué avec 40 cm3 de dichlorométhane et versé sur une suspension de 40 cm3 d'une solution aqueuse sursaturée en hydrogénocarbonate de sodium, maintenue à une température voisine de 0°C. Après dilution avec 10 cm3 d'eau distillée et décantation, la phase aqueuse est réextraite avec deux fois 20 cm3 de diéthyléther. Les phases organiques sont rassemblées, lavées avec 20 cm3 d'eau distillée, 20 cm3 d'une solution aqueuse saturée en chlorure de sodium, séchées sur sulfate de magnésium, filtrées sur sulfate de magnésium et concentrées à sec sous pression réduite (2,7 kPa) à une température voisine de 40°C. On obtient ainsi 370 mg d'une meringue jaune pâle que l'on purifie par chromatographie à pression atmosphérique sur 35 g de silice (0,063-0,2 mm) contenus dans une colonne de 2,5 cm de diamètre en éluant avec un mélange méthanol-dichlorométhane (2-98 en volumes) en recueillant des fractions de 15 cm3. Les fractions ne contenant que le produit cherché sont réunies et concentrées à sec sous pression réduite (2,7 kPa) à 40°C pendant 2 heures. On obtient ainsi 236,2 mg d'acétoxy-4α benzoyloxy-2α époxy-5β,20 trihydroxy-1β,7β,13α éthoxy-10β oxo-9 taxène-11 sous forme d'un solide blanc dont les caractéristiques sont les suivantes :
- spectre de R.M.N. ¹H (400 MHz ; CDCl₃ ; déplacements chimiques δ en ppm ; constantes de couplage J en Hz) : 1,08 (s, 3H : CH₃) ; 1,19 (s, 3H : CH₃) ; 1,29 (t, J = 7,5, 3H : CH₃ éthyle) ; 1,38 (d, J = 9, 1H : OH en 7) ; 1,59 (s, 1H : OH en 1) ; 1,69 (s, 3H : CH₃) ; 1,82 et 2,62 (2 mts, 1H chacun : CH₂ en 6) ; 2,02 (d, J = 5, 1H : OH en 13) ; 2,08 (s, 3H : CH₃) ; 2,30 (s, 3H : COCH₃) ; 2,32 (d, J = 9, 2H : CH₂ en 14) ; 3,56 et 3,67 (2 mts, ¹H chacun : OCH₂ éthyle) ; 3,98 (d, J = 7, 1H : H en 3) ; 4,18 et 4,33 (2 d, J = 8,5 Hz, 1H chacun : CH₂ 20) ; 4,30 (mt, 1H : H 7) ; 4,90 (mt, 1H : H en 13); 4,99 (dd, J = 10 et 1,5, 1H : H en 5) ; 5,05 (s, 1H : H en 10) ; 5,66 (d, J = 7, 1H : H en 2) ; 7,49 (t, J = 7,5, 2H : OCOC₆H₅ H en méta) ; 7,63 (t, J = 7,5, 1H : OCOC₆H₅ H en para) ; 8,12 (d, J = 7,5, 2H : OCOC₆H₅ H en ortho).

L'acétoxy-4α benzoyloxy-2α époxy-5β,20 hydroxy-1β éthoxy-10β oxo-9 bistriéthylsilyloxy-7β,13α taxène-11 (ou éthoxy-10β désacétoxy-10 bistriéthylsilyl-7,13 baccatine III) peut être préparé de la manière suivante :

A une solution de 1 g d'acétoxy-4α benzoyloxy-2α époxy-5β,20 dihydroxy-1β,10β oxo-9 bistriéthylsilyloxy-7β,13α taxène-11 dans 3 cm3 d'iodoéthane et 4cm3 de diméthylformamide, maintenue sous atmosphère d'argon, à une température voisine de 20°C, on ajoute par portions 93 mg d'hydrure de sodium à 50 % en poids dans l'huile de vaseline. La solution est maintenue sous agitation 17 heures à une température voisine de 20°C, puis on ajoute par portions 93 mg d'hydrure de sodium à 50 % en poids dans l'huile de vaseline. Après 50 minutes à une température voisine de 20°C le mélange réactionnel est dilué avec 100 cm3 d'acétate d'éthyle, 10 cm3 d'une solution aqueuse saturée en chlorure d'ammonium. La phase organique décantée est lavée avec six fois 10 cm3 d'eau distillée, puis 10 cm3 d'une solution aqueuse saturée en chlorure de sodium, séchée sur sulfate de magnésium, filtrée sur verre fritté, et concentrée à sec sous pression réduite (2,7 kPa) à une température voisine de 40°C. On obtient ainsi 1,2 g d'une meringue jaune que l'on purifie par chromatographie à pression atmosphérique sur 150 g de silice (0,063-0,2 mm) contenus dans une colonne de 3,5 cm de diamètre en éluant avec un mélange acétate d'éthyle-dichlorométhane (2-98 puis 5-95 en volumes) en recueillant des fractions de 15 cm3. Les fractions ne contenant que les produits cherchés sont réunies et concentrées à sec sous pression réduite (0,27 kPa) à 40°C pendant 2 heures. On obtient ainsi 379,2 mg d'acétoxy-4α benzoyloxy-2α époxy-5β,20 dihydroxy-1β,10β oxo-9 bistriéthylsilyloxy-7β,13α taxène-11 sous forme d'une meringue jaune pâle et 430 mg d'acétoxy-4α benzoyloxy-2α époxy-5β,20 hydroxy-1β éthoxy-10β oxo-9 bistriéthylsilyloxy-7b,13α taxène-11 sous forme d'une meringue blanche dont les caractéristiques sont les suivantes :
- spectre de R.M.N. ¹H (400 MHz ; CDCl₃ ; déplacements chimiques δ en ppm ; constantes de couplage J en Hz) : 0,57 et 0,70 (2 mts, 6H chacun : CH₂ de l'éthyle) ; 0,97 et 1,03 (2 t, J = 7,5, 9H chacun : CH₃ de l'éthyle) ; 1,13 (s, 3H : CH₃) ; 1,20 (s, 3H : CH₃) ; 1,29 (t, J = 7,5, 3H : CH₃ de l'éthoxy en 10) ; 1,58 (s, 1H : OH en 1) ; 1,66 (s, 3H : CH₃) ; 1,89 et 2,58 (2 mts, 1H chacun : CH₂ en 6) ; 2,03 (s, 3H : CH₃) ; 2,13 et 2,23 (2 dd, J =16 et 9, 1H chacun : CH₂ en 14) ; 2,30 (s, 3H: COCH₃) ; 3,53 (mt, 2H : CH₂ de l'éthoxy en 10) ; 3,84 (d, J = 7, 1H : H en 3) ; 4,15 et 4,30 (2 d, J = 8,5, 1H chacun : CH₂ en 20) ; 4,43 (dd, J = 11 et 6,5, 1H : H en 7) ; de 4,90 à 5,00 (mt, 2H : H en 13 et H en 5) ; 5,01 (s, 1H : H en 10) ; 5,61 (d, J = 7, 1H : H en 2) ; 7,48 (t, J = 7,5, 2H : OCOC₆H₅ H en méta) ; 7,61 (t, J = 7,5, 1H : OCOC₆H₅ H en para) ; 8,10 (d, J = 7,5, 2H : OCOC₆H₅ H en ortho).

### EXEMPLE 4

A une suspension contenant 115 mg d'acétoxy-4α benzoyloxy-2α époxy-5β,20 (propyl-1)oxy-10β dihydroxy-1β,13α méthoxy-7β oxo-9 taxène-11, 100 mg d'acide tert-butoxycarbonyl-3 (méthoxy-4 phényl)-2 phényl-4 oxazolidine-1,3 carboxylique-5-(2R,4S,5R) dans 1 cm3 de toluène anhydre, on ajoute successivement, à une température voisine de 20°C, 65 mg de dicyclohexylcarbodiimide, puis 7 mg de N,N'-diméthylamino-4 pyridine. La suspension obtenue est agitée à une température voisine de 20°C sous atmosphère d'argon pendant 1 heure puis purifiée par dépôt direct sur une colonne de chromatographie à pression atmosphérique sur 30 g de silice (0,063-0,2 mm) contenus dans une colonne de 2,5 cm de diamètre (gradient d'élution: acétate d'éthyle-dichlorométhane de 2-98 à 10-90 en volumes) en recueillant des fractions de 10 cm3. Les fractions ne contenant que le produit cherché sont réunies et concentrées à sec sous pression réduite (2,7 kPa) à 40°C pendant 2 heures. On obtient ainsi 276,2 mg d'un solide blanc qui est purifié par chromatographie préparative sur couche mince : 10 plaques préparatives Merck, Kieselgel 60F254, épaisseur 0,5 mm, dépôt en solution dans le dichlorométhane, en éluant par un mélange méthanol-dichlorométhane (3-97 en volumes). Après élution des zones correspondants aux produits principaux par un mélange méthanol-dichlorométhane (15-85 en volumes), filtration sur coton, puis évaporation des solvants sous pression réduite (2,7 kPa) à une température voisine de 40°C, on obtient 84,8 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 (propyl-1)oxy-10β hydroxy-1β méthoxy-7β oxo-9 taxène-11 yle-13α sous forme d'une meringue blanche dont les caractéristiques sont les suivantes :
- spectre de R.M.N. ¹H (300 MHz ; CDCl₃ ; déplacements chimiques δ en ppm ; constantes de couplage J en Hz) : 0,97 (t, J = 7, 3H : CH₃ du propyle) ; 1,07 (s, 9H : C(CH₃)₃) ; 1,19 (s, 6H : CH₃) ; de 1,50 à 1,80 (mt, 3H : OH en 1 et CH₂ central du propyle) ; 1,60 (s, 3H : CH₃) ; 1,70 (s, 3H : CH₃) ; 1,78 et 2,63 (2 mts, 1H chacun : CH₂ en 6) ; 1,82 (mf, 3H : COCH₃) ; 2,07 et 2,19 (2 dd, J = 16 et 9, 1H chacun : CH₂ en 14) ; 3,26 (s, 3H : OCH₃) ; 3,30 et 3,58 (2 mts, 1H chacun : OCH₂ du propyle) ; 3,73 (d, J = 7,5, 1H : H en 3) ; 3,81 (s, 3H : ArOCH₃) ; 3,81 (mt, 1H : H en 7) ; 4,09 et 4,23 (2 d, J = 8,5, 1H chacun : CH₂ en 20) ; 4,57 (d, J = 4,5, 1H : H en 2') ; 4,79 (s, 1H : H en 10); 4,90 (d large, J = 10, 1H : H en 5) ; 5,40 (mf, 1H : H en 3') ; 5,58 (d, J = 7,5, 1H : H en 2) ; 6,13 (t large, J = 9, 1H : H en 13); 6,40 (mf étalé, 1H : H en 5') ; 6,92 (d, J = 8,5, 2H : H aromatiques en ortho du OCH₃) ; de 7,30 à 7,60 (mt, 9H : H aromatiques en 3' - H aromatiques en méta du OCH₃ et OCOC₆H₅ H méta) ; 7,63 (t, J = 7,5, 1H : OCOC₆H₅ H en para) ; 8,03 (d, J = 7,5, 2H : OCOC₆H₅ H en ortho).

Le tert-butoxycarbonylamino-3 hydroxy-2 phényl-3 propionate-(2R,3S) d'acétoxy-4α benzoyloxy-2α époxy-5β,20 (propyl-1)oxy-10β hydroxy-1β méthoxy-7β oxo-9 taxène-11 yle-13α peut être préparé de la manière suivante :

Une solution de 84 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 (propyl-1)oxy-10β hydroxy-1β méthoxy-7β oxo-9 taxène-11 yle-13α dans 0,84 cm3 d'acétate d'éthyle et 0,0071 cm3 d'acide chlorhydrique concentré à 37 % est maintenue sous agitation à une température voisine de 20°C pendant 1 heure sous atmosphère d'argon. Le mélange réactionnel est alors purifié par chromatographie préparative sur couche mince : dépôt du mélange réactionnel brut sur 6 plaques préparatives Merck, Kieselgel 60F254, épaisseur 0,5 mm, en éluant par un mélange méthanol-acétonitrile-dichlorométhane (3-7-90 en volumes). Après élution de la zone correspondant au produit principal par un mélange méthanol-dichlorométhane (15-85 en volumes), filtration sur coton, puis évaporation des solvants sous pression réduite (2,7 kPa) à une température voisine de 40°C, on obtient 27 mg de tert-butoxycarbonylamino-3 hydroxy-2 phényl-3 propionate-(2R,3S) d'acétoxy-4α benzoyloxy-2α époxy-5β,20 (propyl-1)oxy-10β hydroxy-1β méthoxy-7β oxo-9 taxène-11 yle-13α sous forme d'une meringue blanche dont les caractéristiques sont les suivantes :
- spectre de R.M.N ¹H (400 MHz ; CDCl₃ ; déplacements chimiques δ en ppm ; constantes de couplage J en Hz) : 0,99 (t, J = 7, 3H : CH₃ du propyle) ; 1,22 (s, 3H : CH₃) ; 1,25 (s, 3H : CH₃) ; 1,38 (s, 9H : C(CH₃)₃) ; 1,64 (s, 1H : OH en 1) ; 1,69 (mt, 2H : CH₂ central du propyle) ; 1,73 (s, 3H : CH₃) ; 1,80 et 2,70 (2 mts, 1H chacun : CH₂ en 6) ; 1,88 (s, 3H : CH₃) ; 2,30 (mt, 2H : CH₂ en 14) ; 2,38 (s, 3H : COCH₃) ; 3,31 (s, 3H : OCH₃) ; 3,36 et 3,64 (2 mts, 1H chacun : OCH₂ du propyle) ; 3,44 (mf, 1H : OH en 2') ; 3,84 (d, J = 7,5 Hz, 1H : H en 3) ; 3,87 (dd, J = 11 et 6,5, 1H : H en 7) ; 4,18 et 4,30 (2 d, J = 8,5, 1H chacun : CH₂ en 20) ; 4,64 (mt, 1H : H en 2') ; 4,89 (s, 1H : H en 10); 4,98 (d large, J = 10, 1H : H en 5) ; 5,28 (d large, J = 10, 1H : H en 3') ; 5,42 (d, J = 10, 1H : CONH) ; 5,64 (d, J = 7,5, 1H : H en 2) ; 6,22 (t large, J = 9, 1H : H en 13); de 7,25 à 7,45 (mt, 5H : H aromatiques en 3') ; 7,50 (t, J = 7,5, 2H : OCOC₆H₅ H en méta) ; 7,61 (t, J = 7,5, 1H : OCOC₆H₅ H en para) ; 8,12 (d, J = 7,5, 2H : OCOC₆H₅ H en ortho).

L'acétoxy-4α benzoyloxy-2α époxy-5β,20 (propyl-1)oxy-10β dihydroxy-1β,13α méthoxy-7β oxo-9 taxène-11 (ou (propyl-1)oxy-10β méthoxy-7β désacétoxy-10 baccatine III) peut être préparé de la manière suivante :

A une solution de 165 mg d'acétoxy-4α benzoyloxy-2α époxy-5β,20 trihydroxy-1β,7β,13α (propyl-1)oxy-10β oxo-9 taxène-11 dans 1,7 cm3 d'iodométhane et 1 cm3 de diméthylformamide, maintenue sous atmosphère d'argon, à une température voisine de 0°C, on ajoute par portions 30 mg d'hydrure de sodium à 50 % en poids dans l'huile de vaseline. Après 30 minutes à une température voisine de 0°C, le mélange réactionnel est dilué par 40 cm3 d'acétate d'éthyle, 5 cm3 d'eau distillée et 7 cm3 d'une solution aqueuse saturée en chlorure d'ammonium. Après décantation, la phase organique est lavée avec trois fois 7 cm3 d'eau distillée, puis 7 cm3 d'une solution aqueuse saturée en chlorure de sodium, séchée sur sulfate de magnésium, filtrée sur verre fritté, et concentrée à sec sous pression réduite (2,7 kPa) à une température voisine de 40°C. On obtient ainsi 224 mg d'un solide jaune que l'on purifie par chromatographie à pression atmosphérique sur 20 g de silice (0,063-0,2 mm) contenus dans une colonne de 2,5 cm de diamètre (gradient d'élution : acétate d'éthyle-dichlorométhane de 0-100 à 15-85 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 117,5 mg d'acétoxy-4α benzoyloxy-2α époxy-5β,20 (propyl-1)oxy-10β dihydroxy-1β,13α méthoxy-7β oxo-9 taxène-11 sous forme d'une meringue blanche dont les caractéristiques sont les suivantes :
- spectre de R.M.N. ¹H (300 MHz ; CDCl₃ ; déplacements chimiques δ en ppm ; constantes de couplage J en Hz) : 0,98 (t, J = 7, 3H : CH₃ du propyle) ; 1,05 (s, 3H : CH₃) ; 1,19 (s, 3H : CH₃) ; de 1,60 à 1,80 (mt, 2H : CH₂ central du propyle) ; de 1,65 à 1,85 et 2,66 (2 mts, 1H chacun : CH₂ en 6) ; 1,72 (s, 3H : CH₃) ; 2,10 (s, 3H : CH₃) ; de 2,05 à 2,35 (mt, 2H : CH₂ en 14) ; 2,28 (s, 3H : COCH₃) ; 3,32 (s, 3H : OCH₃) ; 3,45 et 3,65 (2 mts, 1H chacun : OCH₂ du propyle) ; 3,92 (d, J = 7,5, 1H : H 3) ; 3,93 (dd, J = 11 et 6, 1H : H en 7) ; 4,16 et 4,32 (2 d, J = 8,5, 1H chacun : CH₂ en 20) ; 4,90 (mt, 1H : H en 13); 4,94 (s, 1H : H en 10) ; 5,03 (d large, J = 10, 1H : H en 5) ; 5,60 (d, J = 7,5, 1H : H en 2) ; 7,48 (t, J = 7,5, 2H : OCOC₆H₅ H en méta) ; 7,62 (t, J = 7,5, 1H : OCOC₆H₅ H en para) ; 8,11 (d, J = 7,5, 2H : OCOC₆H₅ H en ortho).

L'acétoxy-4α benzoyloxy-2α époxy-5β,20 trihydroxy-1β,7β,13α (propyl-1) oxy-10β oxo-9 taxène-11 (ou (propyl-1)oxy-10β désacétoxy-10 baccatine III) peut être préparé de la manière suivante :

A une solution de 585 mg d'acétoxy-4α benzoyloxy-2α époxy-5β,20 hydroxy-1β (propyl-1)oxy-10β oxo-9 bistriéthylsilyloxy-7β,13α taxène-11 dans 6 cm3 de dichlorométhane, maintenue sous atmosphère d'argon, à une température voisine de 20°C, on ajoute 8,75 cm3 de complexe fluorure d'hydrogène-triéthylamine (3HF.Et₃N). Après 24 heures à une température voisine de 20°C, le mélange réactionnel est dilué avec 30 cm3 de dichlorométhane et versé sur une suspension de 30 cm3 d'une solution aqueuse sursaturée en hydrogénocarbonate de sodium, maintenue à une température voisine de 0°C. Après dilution avec 10 cm3 d'eau distillée et décantation, la phase aqueuse est réextraite avec deux fois 20 cm3 de diéthyléther. Les phases organiques sont rassemblées, lavées avec 20 cm3 d'eau distillée, 20 cm3 d'une solution aqueuse saturée en chlorure de sodium, séchées sur sulfate de magnésium, filtrées sur sulfate de magnésium et concentrées à sec sous pression réduite (2,7 kPa) à une température voisine de 40°C. On obtient ainsi 500 mg d'une meringue jaune pâle que l'on purifie par chromatographie à pression atmosphérique sur 40 g de silice (0,063-0,2 mm) contenus dans une colonne de 2,5 cm de diamètre en éluant avec un mélange méthanol-dichlorométhane (2-98 en volumes) en recueillant des fractions de 15 cm3. Les fractions ne contenant que le produit cherché sont réunies et concentrées à sec sous pression réduite (2,7 kPa) à 40°C pendant 2 heures. On obtient ainsi 373,8 mg d'acétoxy-4α benzoyloxy-2α époxy-5β,20 trihydroxy-1β,7β,13α (propyl-1)oxy-10β oxo-9 taxène-11 sous forme d'un solide blanc dont les caractéristiques sont les suivantes :
- spectre de R.M.N. ¹H (300 MHz ; CDCl₃ ; déplacements chimiques δ en ppm ;constantes de couplage J en Hz) : 0,95 (t, J = 7, 3H : CH₃ du propyle) ; 1,06 (s, 3H : CH₃) ; 1,22 (s, 3H : CH₃) ; 1,45 (d, J = 7,5, 1H : OH en 7) ; de 1,60 à 1,80 (mt, 2H : CH₂ central du propyle) ; 1,67 (s, 3H : CH₃) ; 1,83 et 2,62 (2 mts, 1H chacun : CH₂ en 6) ; 2,05 (s, 3H : CH₃) ; 2,05 (mt, 1H : OH en 13); 2,27 (AB limite, 2H : CH₂ en 14) ; 2,28 (s, 3H : COCH₃) ; 3,40 et 3,57 (2 mts, 1H chacun : OCH₂ du propyle) ; 3,97 (d, J = 7,5, 1H : H en 3) ; 4,15 et 4,30 (2 d, J = 8,5, 1H chacun : CH₂ en 20) ; 4,28 (mt, 1H : H en 7) ; 4,90 (mt, 1H : H en 13); 4,98 (d large, J = 10, 1H : H en 5) ; 5,03 (s, 1H : H en 10) ; 5,65 (d, J = 7,5, 1H : H en 2); 7,50 (t, J = 7,5, 2H : OCOC₆H₅ H en méta) ; 7,60 (t, J = 7,5, 1H : OCOC₆H₅ H en para) ; 8,00 (d, J = 7,5, 2H : OCOC₆H₅ H en ortho).

L'acétoxy-4α benzoyloxy-2α époxy-5β,20 hydroxy-1β (propyl-1)oxy-10β oxo-9 bistriéthylsilyloxy-7β,13α taxène-11 (ou (propyl-1)oxy-10β désacétoxy-10 bistriéthylsilyl-7,13 baccatine III) peut être préparé de la manière suivante :

A une solution de 1 g d'acétoxy-4α benzoyloxy-2α époxy-5β,20 dihydroxy-1β,10β oxo-9 bistriéthylsilyloxy-7β,13α taxène-11 dans 3 cm3 d'iodoéthane et 4 cm3 de diméthylformamide, maintenue sous atmosphère d'argon, à une température voisine de 20°C, on ajoute par portions 93 mg d'hydrure de sodium à 50 % en poids dans l'huile de vaseline. La solution est maintenue sous agitation 19 heures à une température voisine de 20°C, puis on ajoute par portions 93 mg d'hydrure de sodium à 50 % en poids dans l'huile de vaseline. Après 3 heures à une température voisine de 20°C le mélange réactionnel est dilué avec 100 cm3 d'acétate d'éthyle, 10 cm3 d'une solution aqueuse saturée en chlorure d'ammonium. La phase organique décantée est lavée avec six fois 10 cm3 d'eau distillée, puis 10 cm3 d'une solution aqueuse saturée en chlorure de sodium, séchée sur sulfate de magnésium, filtrée sur verre fritté, et concentrée à sec sous pression réduite (2,7 kPa) à une température voisine de 40°C. On obtient ainsi 1,32 g d'une meringue jaune pâle que l'on purifie par chromatographie à pression atmosphérique sur 150 g de silice (0,063-0,2 mm) contenus dans une colonne de 3,5 cm de diamètre en éluant avec un mélange acétate d'éthyle-dichlorométhane (2-98 puis 5-95 en volumes) en recueillant des fractions de 15 cm3. Les fractions ne contenant que les produits cherchés sont réunies et concentrées à sec sous pression réduite (0,27 kPa) à 40°C pendant 2 heures. On obtient ainsi 376,3 mg d'acétoxy-4α benzoyloxy-2α époxy-5β,20 dihydroxy-1β,10β oxo-9 bistriéthylsilyloxy-7β,13α taxène-11 sous forme d'une meringue jaune pâle et 395,3 mg d'acétoxy-4α benzoyloxy-2α époxy-5β,20 hydroxy-1β (propyl-1)oxy-10β oxo-9 bistriéthylsilyloxy-7β,13α taxène-11 sous forme d'une meringue jaune pâle dont les caractéristiques sont les suivantes :
- spectre de R.M.N. ¹H (400 MHz ; CDCl₃ ; déplacements chimiques δ en ppm ; constantes de couplage J en Hz) : 0,57 et 0,70 (2 mts, 6H chacun : CH₂ de l'éthyle) ; 0,94 et 1,03 (2 t, J = 7,5, 9H chacun : CH₃ de l'éthyle) ; 0,94 (t, J = 7,5, 3H : CH₃ du propyle) ; 1,14 (s, 3H : CH₃) ; 1,21 (s, 3H : CH₃) ; 1,67 (s, 3H : CH₃) ; 1,69 (mt, 2H : CH₂ central du propyle) ; 1,88 et 2,48 (2 mts, 1H chacun : CH₂ en 6) ; 2,03 (s, 3H : CH₃) ; 2,13 et 2,23 (2 dd, J = 16 et 9, 1H chacun : CH₂ en 14) ; 2,30 (s, 3H : COCH₃) ; 3,40 (mt, 2H : OCH₂ du propyle) ; 3,84 (d, J = 7,5, 1H : H en3) ; 4,16 et 4,30 (2 d, J = 8,5, 1H chacun : CH₂ en 20) ; 4,44 (dd, J = 11 et 6,5, 1H : H en 7) ; 4,96 (d large, J = 10 Hz, 1H : H 5) ; 4.97 (s, 1H : H 10) ; 4,99 (t large, J = 9 Hz, 1H : H en 13); 5,62 (d, J = 7,5, 1H : H en 2); 7,48 (t, J = 7,5, 2H : OCOC₆H₅ H en méta) ; 7,60 (t, J = 7,5, 1H : OCOC₆H₅ H en para) ; 8,10 (d, J = 7,5, 2H : OCOC₆H₅ H en ortho).

Les nouveaux produits de formule générale (I) dans laquelle Z représente un radical de formule générale (II) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants. Cependant, les compositions peuvent aussi se présenter sous forme de comprimés, de pilules, de poudres ou de granulés administrables par voie orale.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérilisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques , des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechlorethamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vindésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées telles que l'hydroxyurée, les dérivés de méthylhydrazine comme la procarbazine, les suppresseurs adrénocorticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogènes comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

## Revendications

1. Taxoïdes de formule générale : 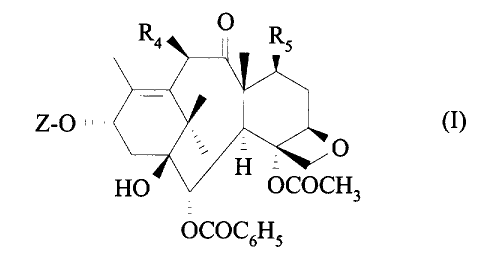 dans laquelle :
Z représente un atome d'hydrogène ou un radical de formule générale : 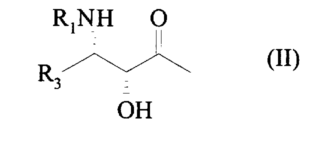 dans laquelle :
R₁ représente
- un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle
- ou un radical R₂-O-CO- dans lequel R₂ représente :
- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,
R₃ représente:
- un radical alcoyle droit ou ramifié contenant 1 à 8 atomes de carbone,
- alcényle droit ou ramifié contenant 2 à 8 atomes de carbone,
- alcynyle droit ou ramifié contenant 2 à 8 atomes de carbone,
- cycloalcoyle contenant 3 à 6 atomes de carbone,
- phényle ou α- ou β-naphtyle
éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, alcoylcarbamoyle, dialcoylcarbamoyle, cyano, nitro et trifluorométhyle,
- ou un hétérocycle aromatique ayant 5 chaînons et contenant un ou plusieurs hétéroatomes, identiques ou différents, choisis parmi les atomes d'azote, d'oxygène ou de soufre et
éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
R₄ représente :
- un radical alcoxy contenant 1 à 6 atomes de carbone en chaîne droite ou ramifiée,
- alcényloxy contenant 3 à 6 atomes de carbone en chaîne droite ou ramifiée,
- alcynyloxy contenant 3 à 6 atomes de carbone en chaîne droite ou ramifiée,
- cycloalcoyloxy contenant 3 à 6 atomes de carbone,
- cycloalcényloxy contenant 4 à 6 atomes de carbone,
ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone,
R₅ représente :
- un radical alcoxy contenant 1 à 6 atomes de carbone en chaîne droite ou ramifiée (éventuellement substitué par un radical alcoxy contenant 1 à 4 atomes de carbone),
- alcényloxy contenant 3 à 6 atomes de carbone,
- alcynyloxy contenant 3 à 6 atomes de carbone,
- cycloalcoyloxy contenant 3 à 6 atomes de carbone,
- cycloalcényloxy contenant 3 à 6 atomes de carbone,
ces radicaux étant éventuellement substitués par un ou plusieurs atomes d'halogène ou par un radical alcoxy contenant 1 à 4 atomes de carbone, alcoylthio contenant 2 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone.

2. Taxoïdes selon la revendication 1 pour lesquels
Z représente un atome d'hydrogène ou un radical de formule générale (II) dans laquelle R₁ représente un radical benzoyle ou un radical R₂-O-CO- dans lequel R₂ représente un radical tert-butyle
et R₃ représente :
- un radical alcoyle contenant 1 à 6 atomes de carbone,
- alcényle contenant 2 à 6 atomes de carbone,
- cycloalcoyle contenant 3 à 6 atomes de carbone,
- phényle
éventuellement substitué par un ou plusieurs atomes ou radicaux, identiques ou différents choisis parmi les atomes d'halogène et les radicaux alcoyles, alcoxy, dialcoylamino, acylamino, alcoxycarbonylamino ou trifluorométhyle
- ou un radical furyle-2 ou -3,
- thiényle-2 ou -3 ou
- thiazolyle-2, -4 ou -5 et
R₄ et R₅, identiques ou différents, représentent chacun un radical alcoyloxy droit ou ramifié contenant 1 à 6 atomes de carbone.

3. Taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ et R₅, identiques ou différents, représentent chacun un radical méthoxy, éthoxy ou propoxy.

4. Procédé de préparation des taxoïdes selon l'une des revendications 1, 2 ou 3 pour lequel Z représente un radical de formule générale (II) caractérisé en ce que l'on estérifie un produit de formule générale : 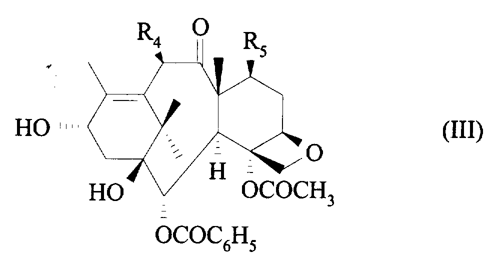 dans laquelle R₄ et R₅ sont définis comme dans l'une des revendications 1, 2 ou 3, au moyen d'un acide de formule générale : 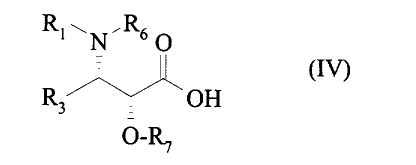 dans laquelle R₁ et R₃ sont définis comme précédemment, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, ou d'un dérivé de cet acide pour obtenir un ester de formule générale : 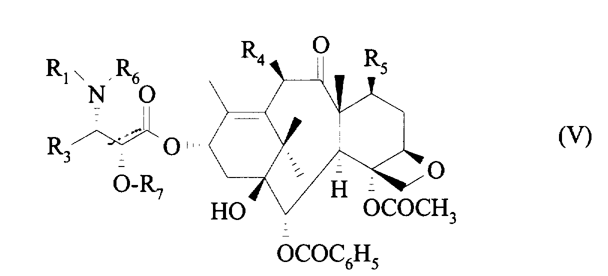 dans laquelle R₁, R₃, R₄, R₅, R₆ et R₇ sont définis comme précédemment, dont on remplace les groupements protecteurs représentés par R₇ et/ou R₆ et R₇ par des atomes d'hydrogène.

5. Procédé selon la revendication 4 caractérisé en ce que l'estérification est effectuée au moyen d'un acide de formule générale (IV) en présence d'un agent de condensation et d'un agent d'activation dans un solvant organique à une température comprise entre -10 et 90°C.

6. Procédé selon la revendication 4 caractérisé en ce que l'estérification est effectuée au moyen d'un acide de formule générale (IV) sous forme d'anhydride symétrique en opérant en présence d'un agent d'activation dans un solvant organique à une température comprise entre 0 et 90°C.

7. Procédé selon la revendication 4 caractérisé en ce que l'estérification est effectuée en utilisant l'acide de formule générale (IV) sous forme d'halogénure ou sous forme d'anhydride mixte avec un acide aliphatique ou aromatique, éventuellement préparé in situ, en présence d'une base en opérant dans un solvant organique à une température comprise entre 0 et 80°C.

8. Procédé selon la revendication 4 caractérisé en ce que l'on remplace les groupements protecteurs R₇ et/ou R₆ et R₇ par des atomes d'hydrogène en opérant, selon leur nature de la manière suivante :
1) lorsque R₆ représente un atome d'hydrogène et R₇ représente un groupement protecteur de la fonction hydroxy, on remplace les groupements protecteurs par des atomes d'hydrogène au moyen d'un acide minéral ou organique utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C, ou au moyen d'une source d'ions fluorures tel qu'un complexe acide fluorhydrique-triéthylamine ou par hydrogénation catalytique,
2) lorsque R₆ et R₇ forment ensemble un hétérocycle saturé à 5 ou 6 chaînons de formule générale : 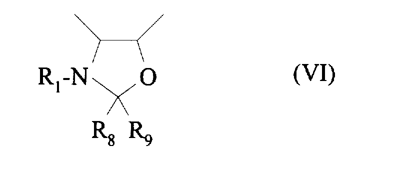 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, on remplace le groupement protecteur formé par R₆ et R₇ par des atomes d'hydrogène en opérant, selon les significations de R₁, R₈ et R₉, de la manière suivante :
a) lorsque R₁ représente un radical tert-butoxycarbonyle, R₈ et R₉, identiques ou différents, représentent un radical alcoyle ou un radical aralcoyle ou aryle, ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, on traite l'ester de formule générale (V) par un acide minéral ou organique éventuellement dans un solvant organique tel qu'un alcool pour obtenir le produit de formule générale : 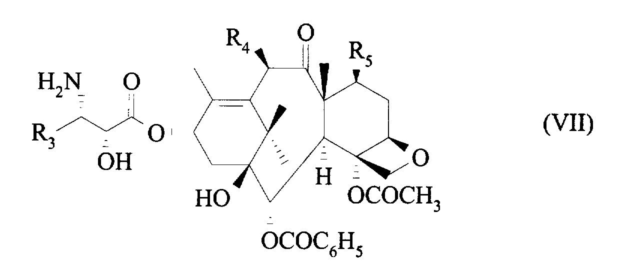 dans laquelle R₃, R₄ et R₅ sont définis comme précédemment, que l'on acyle au moyen de chlorure de benzoyle dans lequel le noyau phényle est éventuellement substitué, de chlorure de thénoyle, de chlorure de furoyle ou d'un produit de formule générale :
R₂-O-CO-X (VIII)
dans laquelle R₂ est défini comme précédemment et X représente un atome d'halogène ou un reste -O-R₂ ou -O-CO-O-R₂, pour obtenir un produit de formule générale (I) dans laquelle Z représente un radical de formule générale (II),
b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, on remplace le groupement protecteur formé par R₆ et R₇ par des atomes d'hydrogène s'effectue en présence d'un acide minéral ou organique utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C.

9. Procédé de préparation d'un nouveau taxoïde selon l'une des revendications 1, 2 ou 3 pour lequel Z représente un atome d'hydrogène, R₄ et R₅ sont définis comme dans l'une des revendications 1, 2 ou 3 caractérisé en ce que l'on traite la 10-désacétyl-baccatine III de formule : 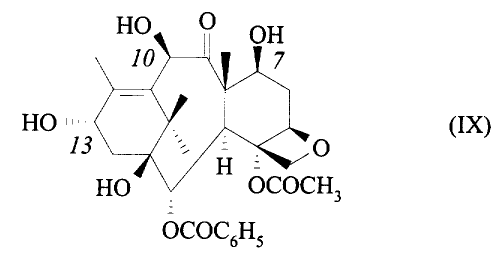 par un halogénure de silyle de formule générale :
(R)₃-Si-Hal (X)
dans laquelle les symboles R, identiques ou différents, représentent un radical alcoyle contenant 1 à 6 atomes de carbone éventuellement substitué par un radical phényle, un radical cycloalcoyle contenant 3 à 6 atomes de carbone ou un radical phényle pour obtenir un produit de formule générale : 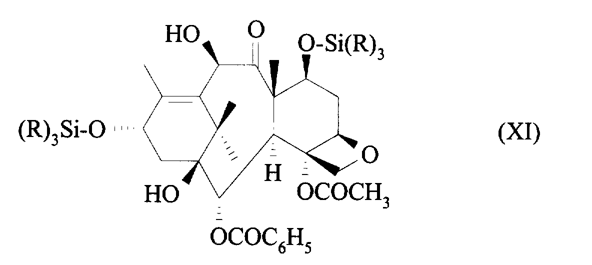 dans laquelle R est défini comme précédemment, que l'on traite par un produit de formule générale :
R_{'4}-X₁ (XII)
dans laquelle R'₄ représente un radical tel que R'₄-O est identique à R₄ défini comme dans l'une des revendications 1, 2 ou 3 et X₁ représente un atome d'halogène ou un reste d'ester réactif pour obtenir un produit de formule générale : 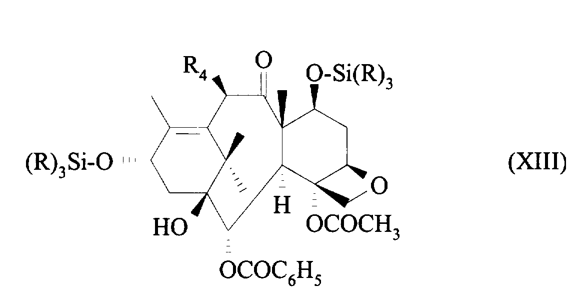 dans laquelle R et R₄ sont définis comme précédemment, dont on remplace les groupements protecteurs silylés par des atomes d'hydrogène pour obtenir un produit de formule générale : 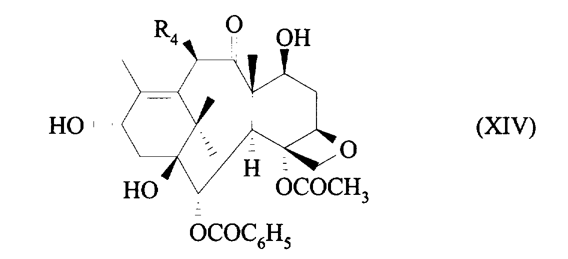 dans laquelle R₄ est défini comme précédemment, qui est éthérifié sélectivement en position 7 par action d'un produit de formule générale :
R'₅-X₂ (XV)
dans laquelle R'₅ représente un radical tel que R'₅-O est identique à R₅ défini comme dans l'une des revendications 1, 2 ou 3 et X₂ représente un reste d'ester réactif ou un atome d'halogène pour donner le produit de formule générale (I) dans laquelle Z représente un atome d'hydrogène.

10. Procédé de préparation d'un produit selon l'une des revendications 1, 2 ou 3 pour lequel Z représente un radical de formule générale (II), R₄ et R₅ sont définis comme dans l'une des revendications 1, 2 ou 3 caractérisé en ce que l'on traite un produit de formule générale : 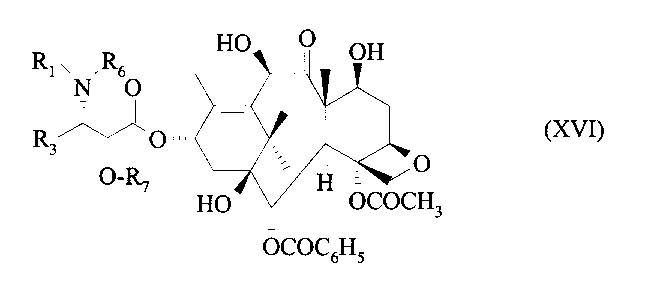 dans laquelle R₁, R₃, R₆ et R₇ sont définis comme dans l'une des revendications 1, 2, 3 ou 4 au moyen d'un produit de formule générale :
(R)₃Si-Hal (X)
dans laquelle les symboles R, identiques ou différents, représentent un radical alcoyle contenant 1 à 6 atomes de carbone, éventuellement substitué par un radical phényle, ou un radical cycloalcoyle contenant 3 à 6 atomes de carbone ou un radical phényle pour obtenir un produit de formule générale : 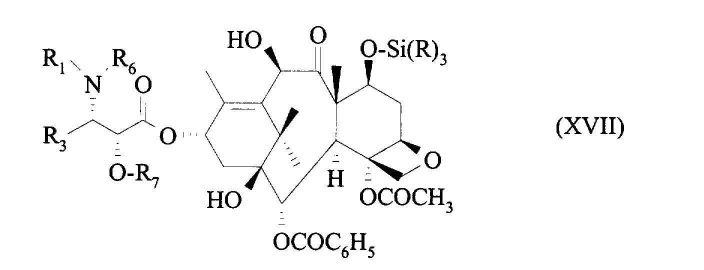 dans laquelle R, R₁, R₃, R₆ et R₇ sont définis comme précédemment, qui est fonctionnalisé en position 10 au moyen d'un produit de formule générale :
R'₄-X₁ (XII)
dans laquelle R'₄ représente un radical tel que R'₄-O est identique à R₄ défini comme dans l'une des revendications 1, 2 ou 3 et X₁ représente un atome d'halogène ou un reste d'ester réactif pour donner un produit de formule générale : 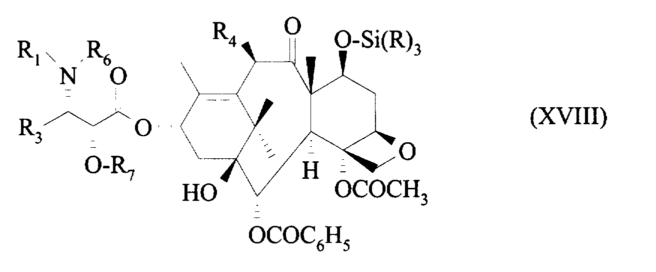 dans laquelle R, R₁, R₃, R₄, R₆ et R₇ sont définis comme précédemment dont le groupement protecteur silylé est remplacé par un atome d'hydrogène pour donner un produit de formule générale : 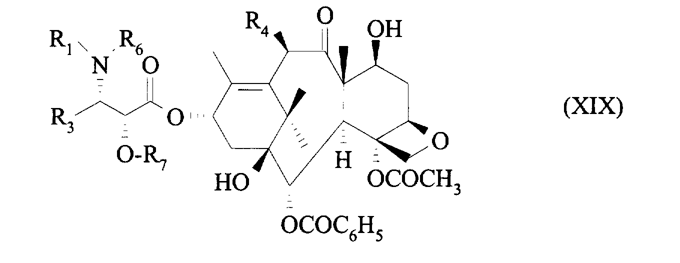 qui, par action d'un produit de formule générale (XV) conduit au produit de formule générale (V) dont les groupements protecteurs sont remplacés par des atomes d'hydrogène pour donner un produit de formule générale (I) dans laquelle Z représente un radical de formule générale (II).

11. Procédé de préparation d'un produit selon l'une des revendications 1, 2 ou 3, caractérisé en ce que l'on fait réagir du nickel Raney activé en présence d'un alcool aliphatique contenant 1 à 3 atomes de carbone ou d'un éther sur un produit de formule générale : 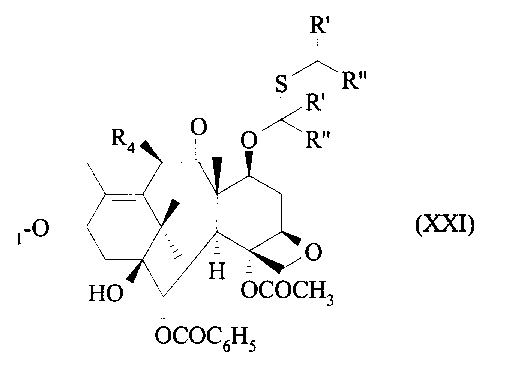 dans laquelle R₄ est défini comme dans l'une des revendications 1,2 ou3, R' et R", identiques ou différents, représentent un atome d'hydrogène ou un radical alcoyle contenant 1 à 6 atomes de carbone, alcényle contenant 2 à 6 atomes de carbone, alcynyle contenant 3 à 6 atomes de carbone, cycloalcoyle contenant 2 à 6 atomes de carbone ou cycloalcényle contenant 3 à 6 atomes de carbone éventuellement substitué, ou bien R' et R" forment ensemble avec l'atome de carbone auquel ils sont liés un radical cycloalcoyle contenant 3 à 6 atomes de carbone ou un radical cycloalcényle contenant 4 à 6 atomes de carbone, et Z₁ représente un atome d'hydrogène ou un radical de formule générale : 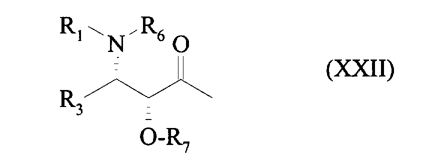 dans laquelle R₁ et R₃ sont définis comme dans l'une des revendications 1 à 3 et R₆ et R₇ sont définis comme dans la revendication 4, pour obtenir un produit de formule générale : 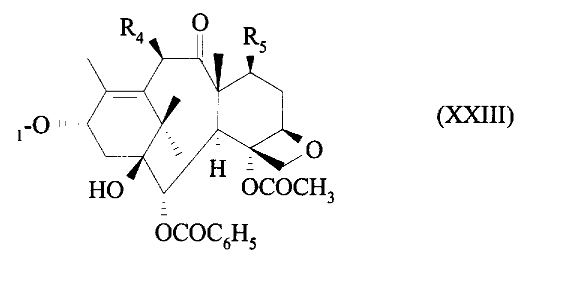 suivi, lorsque Z₁ représente un radical de formule générale (XXII), du remplacement des groupements protecteurs représentés par R₆ et/ou R₆ et R₇ par des atomes d'hydrogène dans les conditions de la revendication 8.

12. Procédé de préparation selon la revendication 11 caractérisé en ce que l'on opére à une température comprise entre -10 et 60°C.

13. Le tert-butoxycarbonylamino-3 hydroxy-2 phényl-3 propionate-(2R,3S) d'acétoxy-4α benzoyloxy-2α époxy-5β,20 hydroxy-1β diméthoxy-7β,10β oxo-9 taxène-11 yle-13α.

14. Le tert-butoxycarbonylamino-3 hydroxy-2 phényl-3 propionate-(2R,3S) d'acétoxy-4α benzoyloxy-2α hydroxy-1β époxy-5β,20 méthoxy-7β éthoxy-10β oxo-9 taxène-11 yle-13α.

15. Le tert-butoxycarbonylamino-3 hydroxy-2 phényl-3 propionate-(2R,3S) d'acétoxy-4α benzoyloxy-2α hydroxy-1β époxy-5β,20 méthoxy-7β (propyl-1)oxy-10β oxo-9 taxène-11 yle-13α.

16. Procédé de préparation du composé selon la revendication 13 caractérisé en ce que,
dans une première étape, on fait réagir le diméthylsulfoxyde sur le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 trihydroxy-1β,7β,10β oxo-9 taxène-11 yle-13α,
dans une deuxième étape on fait réagir sur le produit de la première étape constitué du tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β bis(méthylthiométhoxy)-7β,10β oxo-9 taxène-11 yle-13α une suspension éthanolique de nickel de Raney,
dans une troisième étape on fait réagir sur le produit de la deuxième étape constitué du tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β diméthoxy-7β,10β oxo-9 taxène-11 yle-13α une solution éthanolique d'acide chlorhydrique.

17. Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β bis(méthylthiométhoxy)-7β,10β oxo-9 taxène-11 yle-13α.

18. Composition pharmaceutique caractérisée en ce qu'elle contient au moins un produit selon l'une des revendications 1, 2 ou 3 pour lequel Z représente un radical de formule générale (II) et 13 à 15 en association avec un ou plusieurs diluants ou adjuvants pharmaceutiquement acceptables et éventuellement un ou plusieurs composés compatibles et pharmacologiquement actifs.

19. Composition pharmaceutique caractérisée en ce qu'elle contient au moins le produit selon la revendication 13 en association avec un ou plusieurs diluants ou adjuvants pharmaceutiquement acceptables et éventuellement un ou plusieurs composés compatibles et pharmacologiquement actifs.

20. Composition pharmaceutique caractérisée en ce qu'elle contient au moins le produit selon la revendication 14 en association avec un ou plusieurs diluants ou adjuvants pharmaceutiquement acceptables et éventuellement un ou plusieurs composés compatibles et pharmacologiquement actifs.

21. Composition pharmaceutique caractérisée en ce qu'elle contient au moins le produit selon la revendication 15 en association avec un ou plusieurs diluants ou adjuvants pharmaceutiquement acceptables et éventuellement un ou plusieurs composés compatibles et pharmacologiquement actifs.

## Patentansprüche

1. Taxoide der allgemeinen Formel 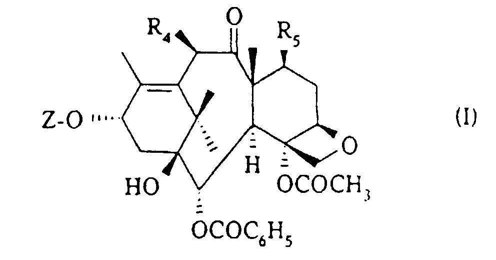 worin
Z ein Wasserstoffatom oder einen Rest der allgemeinen Formel 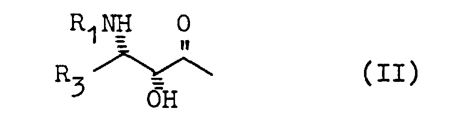 wiedergibt,
worin
R₁ bedeutet
- einen Benzoylrest, gegebenenfalls substituiert durch ein oder mehrere identische oder verschiedene Atome oder Reste, ausgewählt unter den Halogenatomen und den Alkylresten mit 1 bis 4 Kohlenstoffatomen, Alkoxy mit 1 bis 4 Kohlenstoffatomen oder Trifluormethyl, Thenoyl oder Furoyl
- oder einen Rest R₂-O-CO-, worin R₂ bedeutet:
- einen Alkylrest mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen, Bicycloalkyl mit 7 bis 10 Kohlenstoffatomen, wobei diese Reste gegebenenfalls substituiert sind durch einen oder mehrere Substituenten, ausgewählt unter den Halogenatomen und den Hydroxyresten, Alkoxy mit 1 bis 4 Kohlenstoffatomen, Dialkylamino, dessen Alkylteil 1 bis 4 Kohlenstoffatome aufweist, Piperidino, Morpholino, Piperazin-1-yl (gegebenenfalls substituiert in 4-Stellung durch einen Alkylrest mit 1 bis 4 Kohlenstoffatomen oder durch einen Phenylalkylrest, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl (gegebenenfalls substituiert durch ein odermehrere Atome oder Reste, ausgewählt unter den Halogenatomen und den Alkylresten mit 1 bis 4 Kohlenstoffatomen oder Alkoxy mit 1 bis 4 Kohlenstoffatomen), Cyano, Carboxy oder Alkoxycarbonyl, dessen Alkylteil 1 bis 4 Kohlenstoffatome aufweist,
- einen Phenyl- oder α- oder β-Naphthylrest, gegebenenfalls substituiert durch ein oder mehrere Atome oder Reste, ausgewählt unter den Halogenatomen und den Alkylresten mit 1 bis 4 Kohlenstoffatomen oder Alkoxy mit 1 bis 4 Kohlenstoffatomen, oder einen aromatischen heterocyclischen Rest mit 5 Gliedern, ausgewählt vorzugsweise unter den Furyl- und Thienylresten,
- oder einen gesättigten heterocyclischen Rest mit 4 bis 6 Kohlenstoffatomen, gegebenenfalls substituiert durch ein oder mehrere Alkylreste mit 1 bis 4 Kohlenstoffatomen,
R₃ bedeutet:
- einen geradkettigen oder verzweigten Alkylrest mit 1 bis 8 Kohlenstoffatomen,
- geradkettiges oder verzweigtes Alkenyl mit 2 bis 8 Kohlenstoffatomen,
- geradkettiges oder verzweigtes Alkinyl mit 2 bis 8 Kohlenstoffatomen,
- Cycloalkyl mit 3 bis 6 Kohlenstoffatomen,
- Phenyl oder α- oder β-Naphthyl,
gegebenenfalls substituiert durch ein oder mehrere Atome oder Reste, ausgewählt unter den Halogenatomen und den Alkyl-, Alkenyl-, Alkinyl-, Aryl-, Aralkyl-, Alkoxy-, Alkylthio-, Aryloxy-, Arylthio-, Hydroxy-, Hydroxyalkyl-, Mercapto-, Formyl-, Acyl-, Acylamino-, Aroylamino-, Alkoxycarbonylamino-, Amino-, Alkylamino-, Dialkylamino-, Carboxy-, Alkoxycarbonyl-, Carbamoyl-, Alkylcarbamoyl-, Dialkylcarbamoyl-, Cyano-, Nitro- und Trifluormethylresten,
- oder einen aromatischen heterocyclischen Rest mit 5 Gliedern und einem oder mehreren identischen oder verschiedenen Heteroatomen, ausgewählt unter den Stickstoff-, Sauerstoff- oder Schwefelatomen, und
gegebenenfalls substituiert durch ein oder mehrere identische oder verschiedene Substituenten, ausgewählt unter den Halogenatomen und den Alkyl-, Aryl-, Amino-, Alkylamino-, Dialkylamino-, Alkoxycarbonylamino-, Acyl-, Arylcarbonyl-, Cyano-, Carboxy-, Carbamoyl-, Alkylcarbamoyl, Dialkylcarbamoyl- oder Alkoxycarbonylresten, mit der Maßgabe, daß in den Substituenten der Phenyl-, α- oder β-Naphthylreste und der aromatischen heterocyclischen Reste die Alkylreste und Alkylteile der weiteren Reste 1 bis 4 Kohlenstoffatome aufweisen und daß die Alkenyl- und Alkinylreste 2 bis 8 Kohlenstoffatome aufweisen und daß die Arylreste Phenyl- oder α- oder β-Naphthylreste sind,
R₄ bedeutet:
- einen Alkoxyrest mit 1 bis 6 Kohlenstoffatomen in gerader oder verzweigter Kette,
- Alkenyloxy mit 3 bis 6 Kohlenstoffatomen in gerader oder verzweigter Kette,
- Alkinyloxy mit 3 bis 6 Kohlenstoffatomen in gerader oder verzweigter Kette,
- Cycloalkoxy mit 3 bis 6 Kohlenstoffatomen,
- Cycloalkenyloxy mit 4 bis 6 Kohlenstoffatomen,
wobei diese Reste gegebenenfalls substituiert sind durch ein oder mehrere Halogenatome oder durch einen Alkoxyrest mit 1 bis 4 Kohlenstoffatomen, Alkylthio mit 1 bis 4 Kohlenstoffatomen oder einen Carboxyrest, Alkoxycarbonyl, dessen Alkylteil 1 bis 4 Kohlenstoffatome aufweist, Cyano, Carbamoyl, N-Alkylcarbamoyl oder N,N-Dialkylcarbamoyl, deren Alkylteil jeweils 1 bis 4 Kohlenstoffatome enthält oder mit dem Stickstoffatom, an das er gebunden ist, einen gesättigten Heterocyclus mit 5 oder 6 Gliedern und gegebenenfalls einem zweiten Heteroatom bildet, ausgewählt unter den Sauerstoff-, Schwefel- oder Stickstoffatomen, gegebenenfalls substituiert durch einen Alkylrest mit 1 bis 4 Kohlenstoffatomen oder einen Phenyl- oder einen Phenylalkylrest, dessen Alkylteil 1 bis 4 Kohlenstoffatome enthält,
R₅ bedeutet:
- einen Alkoxyrest mit 1 bis 6 Kohlenstoffatomen in gerader oder verzweigter Kette (gegebenenfalls substituiert durch einen Alkoxyrest mit 1 bis 4 Kohlenstoffatomen),
- Alkenyloxy mit 3 bis 6 Kohlenstoffatomen,
- Alkinyloxy mit 3 bis 6 Kohlenstoffatomen,
- Cycloalkoxy mit 3 bis 6 Kohlenstoffatomen,
- Cycloalkenyloxy mit 3 bis 6 Kohlenstoffatomen,
wobei diese Reste gegebenenfalls substituiert sind durch ein oder mehrere Halogenatome oder durch einen Alkoxyrest mit 1 bis 4 Kohlenstoffatomen, Alkylthio mit 2 bis 4 Kohlenstoffatomen oder einen Alkoxyrest, Alkoxycarbonyl, dessen Alkylteil 1 bis 4 Kohlenstoffatome aufweist, Cyano, Carbamoyl, N-Alkylcarbamoyl oder N,N-Dialkylcarbamoyl, deren Alkylteil jeweils 1 bis 4 Kohlenstoffatome enthält oder mit dem Stickstoffatom, an das er gebunden ist, einen gesättigten Heterocyclus mit 5 oder 6 Gliedern und gegebenenfalls einem zweiten Heteroatom bildet, ausgewählt unter Sauerstoff-, Schwefel- oder Stickstoffatomen, gegebenenfalls substituiert durch einen Alkylrest mit 1 bis 4 Kohlenstoffatomen oder einen Phenylrest oder einen Phenylalkylrest, dessen Alkylteil 1 bis 4 Kohlenstoffatome enthält.

2. Taxoide gemäß Anspruch 1, worin
Z ein Wasserstoffatom oder einen Restder allgemeinen Formel (II) bedeutet, worin R₁ einen Benzoylrest oder einen Rest R₂-O-CO- darstellt, worin R₂ einen tert.-Butylrest bedeutet, und
R₅ bedeutet:
- einen Alkylrest mit 1 bis 6 Kohlenstoffatomen,
- Alkenyl mit 2 bis 6 Kohlenstoffatomen,
- Cycloalkyl mit 3 bis 6 Kohlenstoffatomen,
- Phenyl,
gegebenenfalls substituiert durch ein oder mehrere identische oder verschiedene Atome oder Reste, ausgewählt unter den Halogenatomen und den Alkyl-, Alkoxy-, Dialkylamino-, Acylamino-, Alkoxycarbonylamino- oder Trifluormethylresten,
- oder einen Fur-2-yl- oder -3-yl-Rest,
- Thien-2- oder -3-yl oder
- Thiazol-2-yl, -4-yl- oder -5-yl und
R₄ und R₅, identisch oder verschieden, jeweils einen geradkettigen oder verzweigten Alkoxyrest mit 1 bis 6 Kohlenstoffatomen bedeuten.

3. Taxoide gemäß Anspruch 1, worin Z ein Wasserstoffatom oder einen Rest der allgemeinen Formel (II) darstellt, worin R₁ einen Benzoylrest oder einen Rest R₂-O-CO- wiedergibt, worin R₂ einen tert.-Butylrest bedeutet,und R₃ einen Isobutyl-, Isobutenyl-, Butenyl-, Cyclohexyl-, Phenyl-, Fur-2-yl-, Fur-3-yl-, Thien-2-yl-, Thien-3-yl-, Thiazol-2-yl-, Thiazol-4-yl- oder Thiazol-5-yl-Rest bedeutet, R₄ und R₅, identisch oder verschieden, jeweils einen Methoxy-, Ethoxy- oder Propoxyrest wiedergeben.

4. Verfahren zur Herstellung der Taxoide gemäß einem der Ansprüche 1, 2 oder 3, worin Z einen Rest der allgemeinen Formel (II) bedeutet, dadurch gekennzeichnet, daß man ein Produkt der allgemeinen Formel 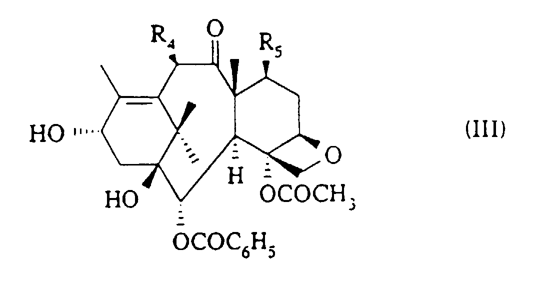 worin R₄ und R₅ wie in einem der Ansprüche 1, 2 oder 3 definiert sind, mit Hilfe einer Säure der allgemeinen Formel 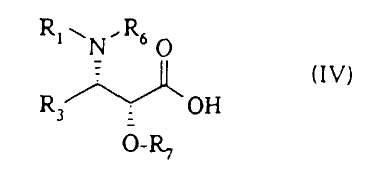 worin R₁ und R₃ wie zuvor definiert sind, R₆ entweder ein Wasserstoffatom bedeutet und R₇ eine Schutzgruppe für die Hydroxyfunktion darstellt oder R₆ und R₇ gemeinsam einen gesättigten Heterocyclus mit 5 oder 6 Gliedern bilden, oder mit Hilfe eines Derivats dieser Säure verestert, um zu einem Ester der allgemeinen Formel 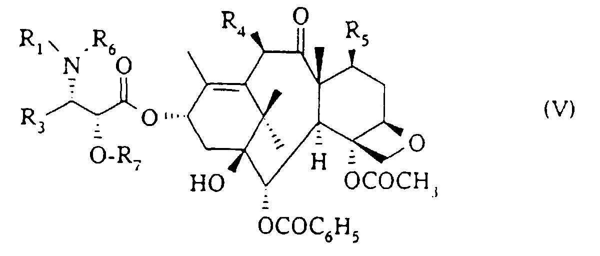 zu gelangen, worin R₁, R₃, R₄, R₅, R₆ und R₇ wie zuvor definiert sind, dessen Schutzgruppen, dargestellt durch R₇ und/ oder R₆ und R₇,durch Wasserstoffatome ersetzt,

5. Verfahren gemäß Anspruch 4, dadurch gekennzeichnet, daß die Veresterung mit Hilfe einer Säure der allgemeinen Formel (IV) in Gegenwart eines Kondensationsmittels und eines Aktivierungsmittels in einem organischen Lösungsmittel bei einer Temperatur zwischen -10 und 90°C durchgeführt wird.

6. Verfahren gemäß Anspruch 4, dadurch gekennzeichnet, daß die Veresterung mit Hilfe einer Säure der allgemeinen Formel (IV) in Form des symmetrischen Anhydrids durchgeführt wird, wobei man in Gegenwart eines Aktivierungsmittels in einem organischen Lösungsmittel bei einer Temperatur zwischen 0 und 90°C arbeitet.

7. Verfahren gemäß Anspruch 4, dadurch gekennzeichnet, daß die Veresterung unter Verwendung der Säure der allgemeinen Formel (IV) in Form des Halogenids oder in Form des gemischten Anhydrids mit einer aliphatischen oder aromatischen Säure, gegebenenfalls hergestellt in situ, in Anwesenheit einer Base durchgeführt wird, wobei man in einem organischen Lösungsmittel bei einer Temperatur zwischen 0 und 80°C arbeitet.

8. Verfahren gemäß Anspruch 4, dadurch gekennzeichnet, daß man die Schutzgruppen R₇ und/oder R₆ und R₇ durch Wasserstoffatome ersetzt, indem man deren Natur entsprechend auf folgende Weise arbeitet:
1) wenn R₆ ein Wasserstoffatom bedeutet und R₇ eine Schutzgruppe für die Hydroxyfunktion darstellt, man die Schutzgruppen durch Wasserstoffatome mit Hilfe einer mineralischen oder organischen Säure, verwendet allein oder im Gemisch, unter Arbeiten in einem organischen Lösungsmittel, ausgewählt unter den Alkoholen, Ethern, Estern, aliphatischen Kohlenwasserstoffen, halogenierten, aliphatischen Kohlenwasserstoffen, aromatischen Kohlenwasserstoffen oder den Nitrilen, bei einer Temperatur zwischen -10 und 60°C oder mit Hilfe einer Quelle für Fluoridionen, wie ein Fluorwasserstoffsäure-Triethylamin-Komplex oder durch katalytische Hydrierung ersetzt,
2) wenn R₆ und R₇ gemeinsam einen gesättigten Heterocyclus mit 5 oder 6 Gliedern der allgemeinen Formel 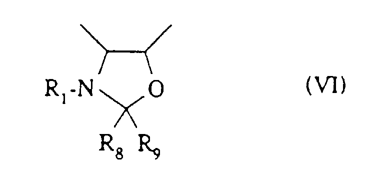 bilden, worin R₁ wie vorstehend definiert ist, R₈ und R₉, identisch oder verschieden, ein Wasserstoffatom oder einen Alkylrest mit 1 bis 4 Kohlenstoffatomen oder einen Aralkylrest, dessen Alkylteil 1 bis 4 Kohlenstoffatome aufweist und dessen Arylteil vorzugsweise einen Phenylrest, gegebenenfalls substituiert durch einen oder mehrere Alkoxyreste mit 1 bis 4 Kohlenstoffatomen, bedeutet, oder einen Arylrest, vorzugsweise in der Bedeutung eines Phenylrestes, gegebenenfalls substituiert durch einen oder mehrere Alkoxyreste mit 1 bis 4 Kohlenstoffatomen, darstellen oder R₈ eine Alkoxygruppe mit 1 bis 4 Kohlenstoffatomen oder einen Trihalomethylrest, wie einen Trichlormethylrest, oder einen Phenylrest, substituiert durch einen Trihalomethylrest, wie einen Trichlormethylrest, bedeutet und R₉ ein Wasserstoffatom darstellt oder R₈ und R₉ gemeinsam mit dem Kohlenstoffatom, an das sie gebunden sind, einen Ring mit 4 bis 7 Gliedern bilden, man die durch R₆ und R₇ gebildete Schutzgruppe durch Wasserstoffatome austauscht, indem man entsprechend den Bedeutungen von R₁, R₈ und R₉ wie folgt arbeitet:
a) wenn R₁ einen tert.-Butoxycarbonylrest bedeutet, R₈ und R₉, identisch oder verschieden, einen Alkylrest oder einen Aralkylrest oder einen Arylrest wiedergeben oder R₈ einen Trihalomethylrest oder einen Phenylrest, substituiert durch einen Trihalomethylrest, bedeutet und R₉ ein Wasserstoffatom darstellt oder R₈ und R₉ gemeinsam einen Ring mit 4 bis 7 Gliedern bilden, man den Ester der allgemeinen Formel (V) mit einer mineralischen oder organischen Säure, gegebenenfalls in einem organischen Lösungsmittel, wie einem Alkohol, behandelt, um zu dem Produkt der allgemeinen Formel 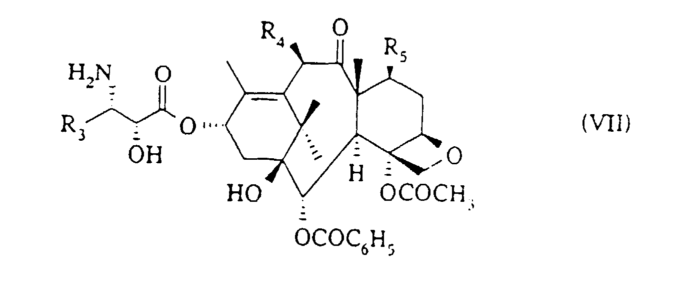 zu gelangen, worin R₃, R₄ und R₅ wie vorstehend definiert sind, welches man mit Hilfe von Benzoylchlorid, worin der Phenylkern gegebenenfalls substituiert ist, von Thenoylchlorid, von Furoylchlorid oder mit Hilfe eines Produkts der allgemeinen Formel
R₂-O-CO-X (VIII)
worin R₂ wie vorstehend definiert ist und X ein Halogenatom oder einen Rest -O-R₂ oder -O-CO-O-R₂ wiedergibt, acyliert, um zu einem Produkt der allgemeinen Formel (I) zu gelangen, worin Z für einen Rest der allgemeinen Formel (II) steht,
b) wenn R₁ einen gegebenenfalls substituierten Benzoylrest, Thenoyl- oder Furoylrest oder einen Rest R₂O-CO-, worin R₂ wie vorstehend definiert ist, darstellt, R₈ ein Wasserstoffatom oder einen Alkoxyrest mit 1 bis 4 Kohlenstoffatomen oder einen Phenylrest, substituiert durch einen oder mehrere Alkoxyreste mit 1 bis 4 Kohlenstoffatomen, bedeutet und R₉ ein Wasserstoffatom darstellt, der Austausch der durch R₆ und R₇ gebildeten Schutzgruppe durch Wasserstoffatome in Anwesenheit einer mineralischen oder organischen Säure, verwendet allein oder im Gemisch in stöchiometrischer oder katalytischer Menge, erfolgt, wobei man in einem organischen Lösungsmittel, ausgewählt unter den Alkoholen, den Ethern, den Estern, den aliphatischen Kohlenwasserstoffen, den halogenierten, aliphatischen Kohlenwasserstoffen und den aromatischen Kohlenwasserstoffen, bei einer Temperatur zwischen -10 und 60°C, vorzugsweise zwischen 15 und 30°C, arbeitet.

9. Verfahren zur Herstellung eines neuen Taxoids gemäß einem der Ansprüche 1, 2 oder 3, worin Z ein Wasserstoffatom bedeutet, R₄ und R₅ wie in einem der Ansprüche 1, 2 oder 3 definiert sind, dadurch gekennzeichnet, daß man 10-Desacetyl-baccatin-III der Formel 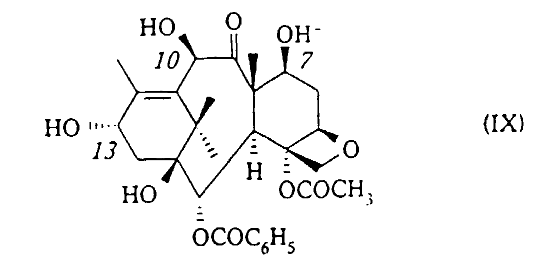 mit einem Silylhalogenid der allgemeinen Formel
(R)₃-Si-Hal (X)
worin die Symbole R, identisch oder verschieden, einen gegebenenfalls durch einen Phenylrest substituierten Alkylrest mit 1 bis 6 Kohlenstoffatomen, einen Cycloalkylrest mit 3 bis 6 Kohlenstoffatomen oder einen Phenylrest bedeuten, behandelt, um zu einem Produkt der allgemeinen Formel 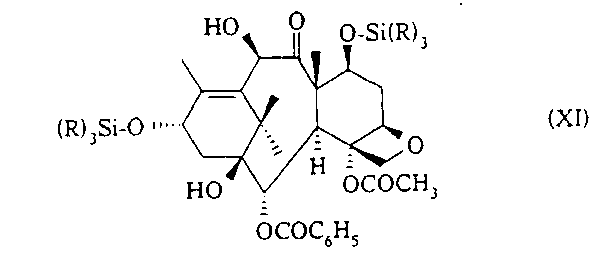 zu gelangen, worin R wie vorstehend definiert ist, welches man mit einem Produkt der allgemeinen Formel
R'₄-X₁ (XII)
behandelt, worin R'₄ einen Rest darstellt, derart, daß R'₄-O mit R₄, wie in einem der Ansprüche 1, 2 oder 3 definiert, identisch ist und X₁ ein Halogenatom oder einen Rest eines reaktiven Esters darstellt, um zu einem Produkt der allgemeinen Formel 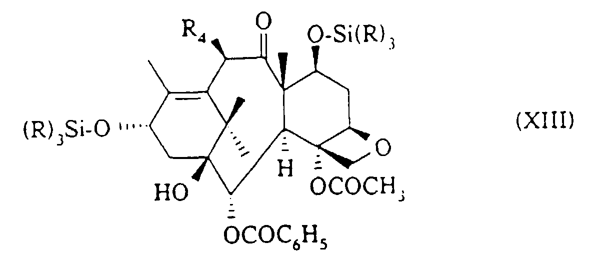 zu gelangen, worin R und R₄ wie vorstehend definiert sind, dessen Silylschutzgruppen man durch Wasserstoffatome ersetzt, um zu einem Produkt der allgemeinen Formel 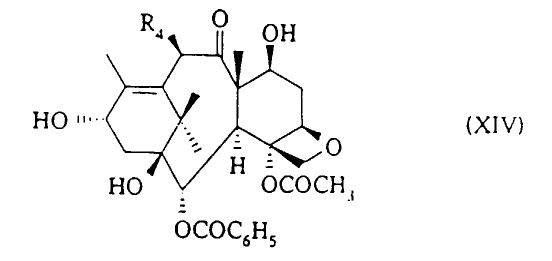 zu gelangen, worin R₄ wie vorstehend definiert ist, welches selektiv in 7-Stellung durch Umsetzung mit einem Produkt der allgemeinen Formel
R'₅-X₂ (XV)
verethert wird, worin R'₅ einen Rest darstellt, derart, daß R'₅-O mit R₅, wie in einem der Ansprüche 1,2 oder 3 definiert, identisch ist, und X₂ einen reaktiven Ester oder ein Halogenatom bedeutet, um zu dem Produkt der allgemeinen Formel (I) zu gelangen, worin Z ein Wasserstoffatom darstellt.

10. Verfahren zur Herstellung eines Produkts gemäß einem der Ansprüche 1, 2 oder 3, worin Z einen Rest der allgemeinen Formel (II) darstellt, R₄ und R₅ wie in einem der Ansprüche 1, 2 oder 3 definiert sind, dadurch gekennzeichnet, daß man ein Produkt der allgemeinen Formel 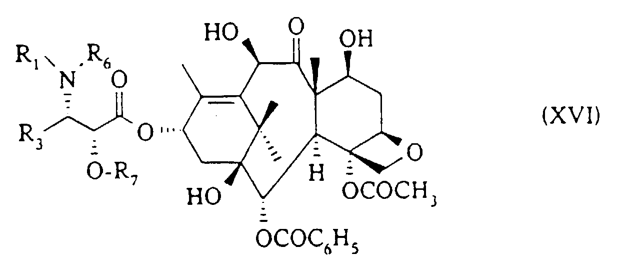 worin R₁, R₃, R₆ und R₇ wie in einem der Ansprüche 1, 2, 3 oder 4 definiert sind, mit Hilfe eines Produkts der allgemeinen Formel
(R)₃Si-Hal (X)
behandelt, worin die Symbole R, identisch oder verschieden, einen gegebenenfalls durch einen Phenylrest substituierten Alkylrest mit 1 bis 6 Kohlenstoffatomen oder einen Cycloalkylrest mit 3 bis 6 Kohlenstoffatomen oder einen Phenylrest bedeuten, um zu einem Produkt der allgemeinen Formel 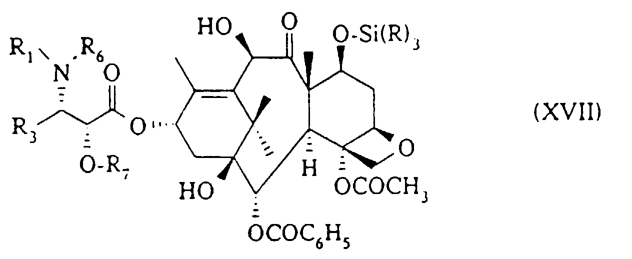 zu gelangen, worin R, R₁, R₃, R₆ und R₇ wie zuvor definiert sind, welches in 10-Stellung mit Hilfe eines Produkts der allgemeinen Formel
R'₄-X₁ (XII)
funktionalisiert wird, worin R'₄ einen Rest darstellt, derart, daß R'₄-O mit R₄, wie in einem der Ansprüche 1, 2 oder 3 definiert, identisch ist und X₁ ein Halogenatom oder einen Rest eines reaktiven Esters wiedergibt, um zu einem Produkt der allgemeinen Formel 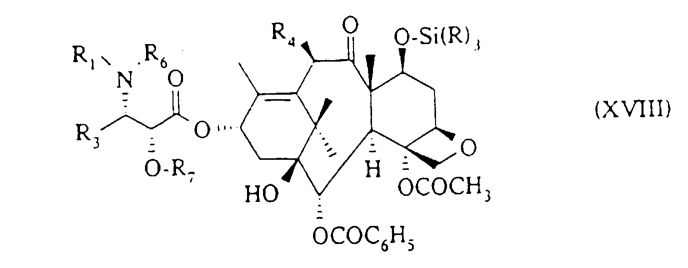 zu gelangen, worin R, R₁, R₃, R₄, R₆ und R₇ wie zuvor definiert sind, dessen Silylschutzgruppe man durch ein Wasserstoffatom ersetzt, um zu einem Produkt der allgemeinen Formel 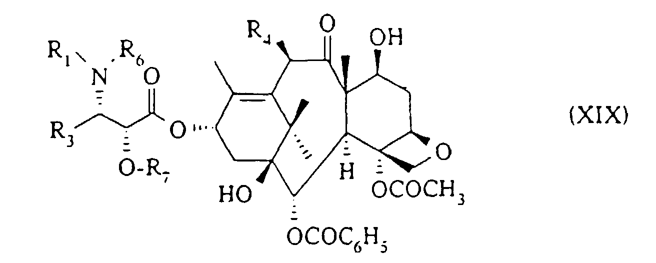 zu gelangen, welches durch Umsetzung mit einem Produkt der allgemeinen Formel (XV) zu einem Produkt der allgemeinen Formel (V) führt, dessen Schutzgruppen durch Wasserstoffatome ersetzt werden, um zu einem Produkt der allgemeinen Formel (I) zu gelangen, worin Z einen Rest der allgemeinen Formel (II) bedeutet.

11. Verfahren zur Herstellung eines Produkts gemäß einem der Ansprüche 1, 2 oder 3, dadurch gekennzeichnet, daß man aktiviertes Raneynickel in Gegenwart eines aliphatischen Alkohols mit 1 bis 3 Kohlenstoffatomen oder eines Ethers mit einem Produkt der allgemeinen Formel 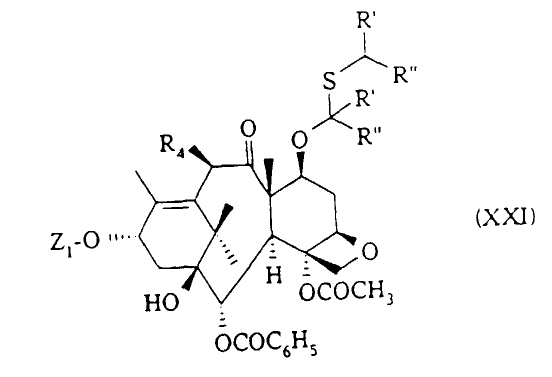 umsetzt, worin R₄ wie in einem der Ansprüche 1, 2 oder 3 definiert ist, R' und R", identisch oder verschieden, ein Wasserstoffatom oder einen Alkylrest mit 1 bis 6 Kohlenstoffatomen, Alkenyl mit 2 bis 6 Kohlenstoffatomen, Alkinyl mit 3 bis 6 Kohlenstoffatomen, Cycloalkyl mit 2 bis 6 Kohlenstoffatomen oder Cycloalkenyl mit 3 bis 6 Kohlenstoffatomen, gegebenenfalls substituiert, bedeuten oder R' und R" gemeinsam mit dem Kohlenstoffatom, an das sie gebunden sind, einen Cycloalkylrest mit 3 bis 6 Kohlenstoffatomen oder einen Cycloalkenylrest mit 4 bis 6 Kohlenstoffatomen bilden, und Z₁ ein Wasserstoffatom oder einen Rest der allgemeinen Formel 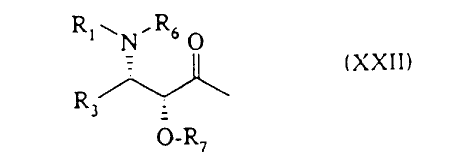 darstellt, worin R₁ und R₃ wie in einem der Ansprüche 1 bis 3 definiert sind und R₆ und R₇ wie in Anspruch 4 definiert sind, um zu einem Produkt der allgemeinen Formel 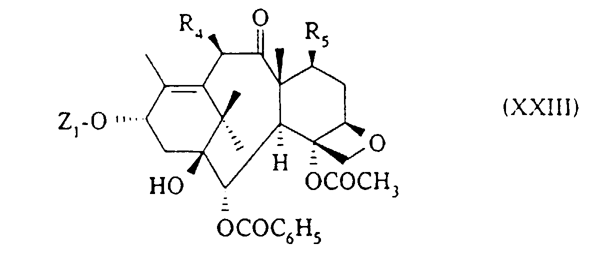 zu gelangen, woran, wenn Z₁ einen Rest der allgemeinen Formel (XXII) bedeutet, sich der Austausch der durch R₆ und/ oder R₆ und R₇ dargestellten Schutzgruppen durch Wasserstoffatome unter den Bedingungen von Anspruch 8 anschließt.

12. Verfahren gemäß Anspruch 11, dadurch gekennzeichnet, daß man bei einer Temperatur zwischen -10 und 60°C arbeitet.

13. 4α-Acetoxy-2α-benzoyloxy-5β,20-epoxy-1β-hydroxy-7β, 10β-dimethoxy-9-oxo-11-taxen-13α-yl-(2R,3S)-3-tert.-butoxycarbonylamino-2-hydroxy-3-phenylpropionat.

14. 4α-Acetoxy-2α-benzoyloxy-1β-hydroxy-5β,20-epoxy-7β-methoxy-10β-ethoxy-9-oxo-11-taxen-13α-yl-(2R,3S)-3-tert.-butoxycarbonylamino-2-hydroxy-3-phenyl-propionat.

15. 4α-Acetoxy-2α-benzoyloxy-1β-hydroxy-5β,20-epoxy-7β-methoxy- 10β-(prop-1-yl) -oxy-9-oxo-11-taxen-13α-yl-(2R,3S)-3-tert.-butoxycarbonylamino-2-hydroxy-3-phenylpropionat.

16. Verfahren zur Herstellung der Verbindung gemäß Anspruch 13, dadurch gekennzeichnet, daß man
in einer ersten Stufe das Dimethylsulfoxid mit 4α-Acetoxy-2α-benzoyloxy-5β,20-epoxy-1β,7β,10β-trihydroxy-9-oxo-11-taxen-13α-yl-(2R,4S,5R)-3-tert.-butoxycarbonyl-2(4-methoxyphenyl)-4-phenyl-1,3-oxazolidin-5-carboxylat umsetzt,
in einer zweiten Stufe mit dem Produkt der ersten Stufe, bestehend aus 4α-Acetoxy-2α-benzoyloxy-5β,20-epoxy-1β-hydroxy-7β,10β-bis-(methylthiomethoxy)-9-oxo-11-taxen-13α-yl-(2R,4S,5R)-3-tert.-butoxycarbonyl-2-(4-methoxyphenyl)-4-phenyl-1,3-oxazolidin-5-carboxylat, eine ethanolische Suspension von Raneynickel umsetzt,
in einer dritten Stufe mit dem Produkt der zweiten Stufe, bestehend aus 4α-Acetoxy-2α-Benzoyloxy-5β,20-epoxy-1β-hydroxy-7β,10β-dimethoxy-9-oxo-11-taxen-13α-yl-(2R,4S,5R)-3-tert.-butoxycarbonyl-2-(4-methoxyphenyl)-4-phenyl-1,3-oxazolidin-5-carboxylat, eine ethanolische Chlorwasserstoffsäure-Lösung umsetzt.

17. 4α-Acetoxy-2α-benzoyloxy-5β,20-epoxy-1β-hydroxy-7β,10β-bis-(methylthiomethoxy)-9-oxo-11-taxen-13α-yl-(2R, 4S,5R)-3-tert.-butoxycarbonyl-2-(4-methoxyphenyl)-4-phenyl-1,3-oxazolidin-5-carboxylat.

18. Pharmazeutische Zusammensetzung, dadurch gekennzeichnet, daß sie zumindest ein Produkt gemäß einem der Ansprüche 1, 2 oder 3, worin Z einen Rest der allgemeinen Formel (II) bedeutet, und 13 bis 15 zusammen mit einem oder mehreren pharmazeutisch verträglichen Verdünnungsmitteln oder Adjuvantien und gegebenenfalls einer oder mehreren verträglichen und pharmazeutisch aktiven Verbindungen enthält.

19. Pharmazeutische Zusammensetzung, dadurch gekennzeichnet, daß sie zumindest das Produkt gemäß Anspruch 13 gemeinsam mit einem oder mehreren pharmazeutisch verträglichen Verdünnungsmitteln oder Adjuvantien und gegebenenfalls einer oder mehreren verträglichen und pharmakologisch aktiven Verbindungen enthält.

20. Pharmazeutische Zusammensetzung, dadurch gekennzeichnet, daß sie zumindest das Produkt gemäß Anspruch 14 gemeinsam mit einem oder mehreren pharmazeutisch verträglichen Verdünnungsmitteln oder Adjuvantien und gegebenenfalls einer oder mehreren verträglichen und pharmakologisch aktiven Verbindungen enthält.

21. Pharmazeutische Zusammensetzung, dadurch gekennzeichnet, daß sie zumindest das Produkt gemäß Anspruch 15 gemeinsam mit einem oder mehreren pharmazeutisch verträglichen Verdünnungsmitteln oder Adjuvantien und gegebenenfalls einer oder mehreren verträglichen und pharmakologisch aktiven Verbindungen enthält.

## Claims

1. Taxoids of general formula: 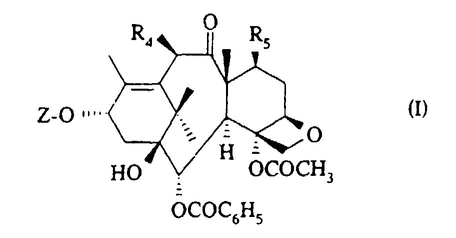 in which:
z represents a hydrogen atom or a radical of general formula: 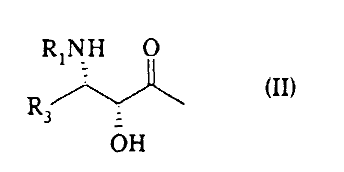 in which:
R₁ represents
- a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals, a thenoyl or furoyl radical
- or a radical R₂-O-CO- in which R₂ represents:
- an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,
R₃ represents:
- an unbranched or branched alkyl radical containing 1 to 8 carbon atoms,
- an unbranched or branched alkenyl radical containing 2 to 8 carbon atoms,
- an unbranched or branched alkynyl radical containing 2 to 8 carbon atoms,
- a cycloalkyl radical containing 3 to 6 carbon atoms,
- a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals,
- or a 5-membered aromatic heterocycle containing one or more identical or different hetero atoms chosen from nitrogen, oxygen and sulphur atoms and
optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals, on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals,
R₄ represents:
- an alkoxy radical containing 1 to 6 carbon atoms in an unbranched or branched chain,
- an alkenyloxy radical containing 3 to 6 carbon atoms in an unbranched or branched chain,
- an alkynyloxy radical containing 3 to 6 carbon atoms in an unbranched or branched chain,
- a cycloalkyloxy radical containing 3 to 6 carbon atoms
- a cycloalkenyloxy radical containing 4 to 6 carbon atoms,
these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms,
R₅ represents:
- an alkoxy radical containing 1 to 6 carbon atoms in an unbranched or branched chain (optionally substituted with an alkoxy radical containing 1 to 4 carbon atoms),
- an alkenyloxy radical containing 3 to 6 carbon atoms,
- an alkynyloxy radical containing 3 to 6 carbon atoms,
- a cycloalkyloxy radical containing 3 to 6 carbon atoms,
- a cycloalkenyloxy radical containing 3 to 6 carbon atoms,
these radicals being optionally substituted with one or more halogen atoms or with an alkoxy radical containing 1 to 4 carbon atoms, an alkylthio radical containing 2 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms.

2. Taxoids according to claim 1 for which
Z represents a hydrogen atom or a radical of general formula (II) in which R₁ represents a benzoyl radical or a radical R₂-O-CO- in which R₂ represents a tert-butyl radical
and R₃ represents:
- an alkyl radical containing 1 to 6 carbon atoms,
- an alkenyl radical containing 2 to 6 carbon atoms,
- a cycloalkyl radical containing 3 to 6 carbon atoms,
- a phenyl radical optionally substituted with one or more identical or different atoms or radicals chosen from halogen atoms and alkyl, alkoxy, dialkylamino, acylamino, alkoxycarbonylamino or trifluoromethyl radical,
- or a 2- or 3-furyl,
- 2- or 3-thienyl or
- 2-, 4- or 5-thiazolyl radical, and
R₄ and R₅, which may be identical or different, each represent an unbranched or branched alkyloxy radical containing 1 to 6 carbon atoms.

3. Taxoids according to claim 1 for which Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, and R₄ and R₅, which may be identical or different, each represent a methoxy, ethoxy or propoxy radical.

4. Process for preparing the taxoids according to one of claims 1, 2 and 3 for which Z represents a radical of general formula (II), characterized in that a product of general formula: 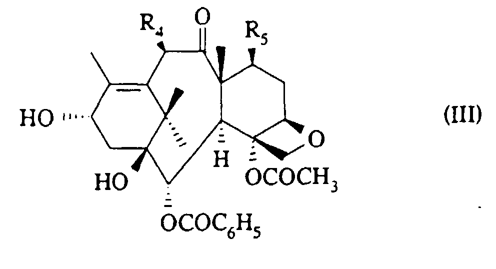 in which R₄ and R₅ are defined as in one of claims 1, 2 and 3, is esterified by means of an acid of general formula: 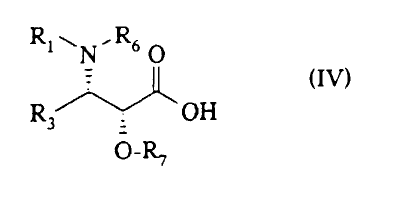 in which R₁ and R₃ are defined as above, and either R6 represents a hydrogen atom and R₇ represents a group protecting the hydroxyl function, or R₆ and R₇ together form a saturated 5- or 6-membered heterocycle, or by means of a derivative of this acid, to obtain an ester of general formula: 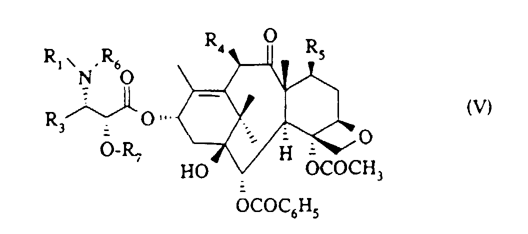 in which R₁, R₃, R₄, R₅, R₆ and R₇ are defined as above, the protective groups of which, represented by R₇ and/or R₆ and R₇, are replaced by hydrogen atoms.

5. Process according to claim 4, characterized in that the esterification is performed by means of an acid of general formula (IV) in the presence of a condensing agent and an activating agent in an organic solvent at a temperature of between -10 and 90°C.

6. Process according to claim 4, characterized in that the esterification is performed by means of an acid of general formula (IV) in the form of the symmetrical anhydride, working in the presence of an activating agent in an organic solvent at a temperature of between 0 and 90°C.

7. Process according to claim 4, characterized in that the esterification is performed using the acid of general formula (IV) in halide form or in the form of a mixed anhydride with an aliphatic or aromatic acid, optionally prepared in situ, in the presence of a base, working in an organic solvent at a temperature of between 0 and 80°C.

8. Process according to claim 4, characterized in that the protective groups R₇ and/or R₆ and R₇ are replaced by hydrogen atoms, working, depending on their nature, in the following manner:
1) when R₆ represents a hydrogen atom and R₇ represents a group protecting the hydroxyl function, the protective groups are replaced by hydrogen atoms by means of an inorganic or organic acid used alone or mixed, working in an organic solvent chosen from alcohols, ethers, esters, aliphatic hydrocarbons, halogenated aliphatic hydrocarbons, aromatic hydrocarbons or nitriles at a temperature of between -10 and 60°C, or by means of a source of fluoride ions such as a hydrofluoric acid/triethylamine complex, or by catalytic hydrogenation,
2) when R₆ and R₇ together form a saturated 5- or 6-membered heterocycle of general formula: 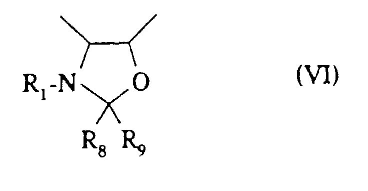 in which R₁ is defined as above and R₈ and R₉, which may be identical or different, represent a hydrogen atom or an alkyl radical containing 1 to 4 carbon atoms, or an aralkyl radical in which the alkyl portion contains 1 to 4 carbon atoms and the aryl portion preferably represents a phenyl radical optionally substituted with one or more alkoxy radicals containing 1 to 4 carbon atoms, or an aryl radical preferably representing a phenyl radical optionally substituted with one or more alkoxy radicals containing 1 to 4 carbon atoms, or alternatively R₈ represents an alkoxy radical containing 1 to 4 carbon atoms or a trihalomethyl radical such as trichloromethyl or a phenyl radical substituted with a trihalomethyl radical such as trichloromethyl and R₉ represents a hydrogen atom, or alternatively R₈ and R₉, together with the carbon atom to which they are linked, form a 4- to 7-membered ring, the protective group formed by R₆ and R₇ is replaced by hydrogen atoms, working, depending on the meanings of R₁, R₈ and R₉, in the following manner:
a) when R₁ represents a tert-butoxycarbonyl radical and R₈ and R₉, which may be identical or different, represent an alkyl radical or an aralkyl or aryl radical, or alternatively R₈ represents a trihalomethyl radical or a phenyl radical substituted with a trihalomethyl radical and R₉ represents a hydrogen atom, or alternatively R₈ and R₉ together form a 4- to 7-membered ring, the ester of general formula (V) is treated with an inorganic or organic acid, where appropriate in an organic solvent such as an alcohol, to obtain the product of general formula: 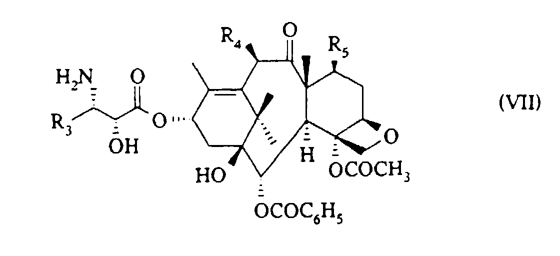 in which R₃, R₄ and R₅ are defined as above, which is acylated by means of benzoyl chloride in which the phenyl ring is optionally substituted or by means of thenoyl chloride, of furoyl chloride or of a product of general formula:
R₂-O-CO-X (VIII)
in which R₂ is defined as above and X represents a halogen atom or a residue -O-R₂ or -O-CO-O-R₂, to obtain a product of general formula (I) in which Z represents a radical of general formula (II),
b) when R₁ represents an optionally substituted benzoyl radical, a thenoyl or furoyl radical or a radical R₂O-CO- in which R₂ is defined as above, R₈ represents a hydrogen atom or an alkoxy radical containing 1 to 4 carbon atoms or a phenyl radical substituted with one or more alkoxy radicals containing 1 to 4 carbon atoms and R₉ represents a hydrogen atom, the protective group formed by R₆ and R₇ is replaced by hydrogen atoms, working in the presence of an inorganic or organic acid used alone or mixed in a stoichiometric or catalytic amount, and working in an organic solvent chosen from alcohols, ethers, esters, aliphatic hydrocarbons, halogenated aliphatic hydrocarbons and aromatic hydrocarbons at a temperature of between -10 and 60°C, and preferably between 15 and 30°C.

9. Process for preparing a new taxoid according to one of claims 1, 2 and 3 for which Z represents a hydrogen atom and R₄ and R₅ are defined as in one of claims 1, 2 and 3, characterized in that 10-deacetylbaccatin III of formula: 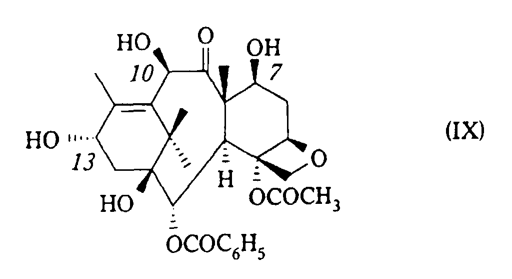 is treated with a silyl halide of general formula:
(R)₃-Si-Hal (X)
in which the symbols R, which may be identical or different, represent an alkyl radical containing 1 to 6 carbon atoms, optionally substituted with a phenyl radical, a cycloalkyl radical containing 3 to 6 carbon atoms or a phenyl radical, to obtain a product of general formula: 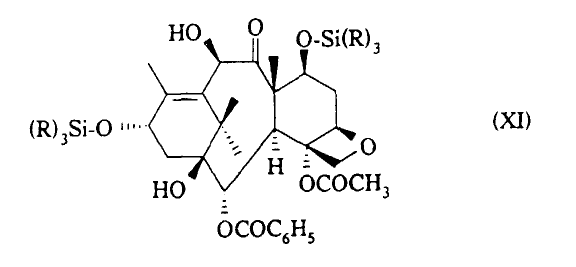 in which R is defined as above, which is treated with a product of general formula:
R'₄-X₁ (XII)
in which R'₄ represents a radical such that R'₄-O is identical to R₄ defined as in one of claims 1, 2 and 3 and X₁ represents a halogen atom or a reactive ester residue, to obtain a product of general formula: 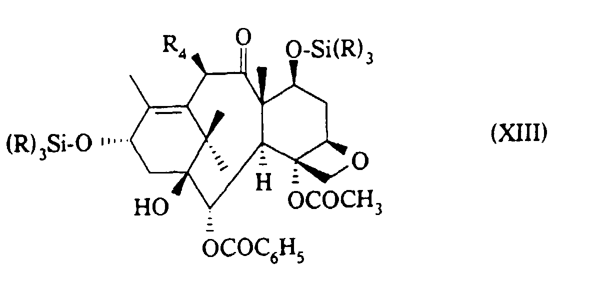 in which R and R₄ are defined as above, the silyl protective groups of which are replaced by hydrogen atoms to obtain a product of general formula: 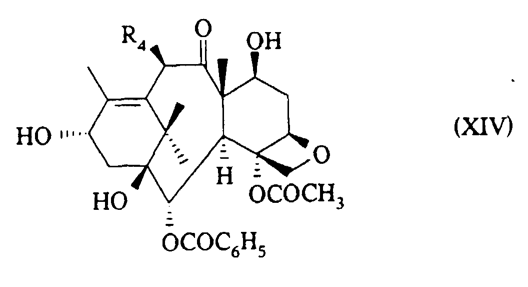 in which R₄ is defined as above, which is etherified selectively at position 7 by the action of a product of general formula:
R'₅-X₂ (XV)
in which R'₅ represents a radical such that R'₅-O is identical to R₅ defined as in one of claims 1, 2 and 3 and X₂ represents a reactive ester residue or a halogen atom, to give the product of general formula (I) in which Z represents a hydrogen atom.

10. Process for preparing a product according to one of claims 1, 2 and 3 for which Z represents a radical of general formula (II) and R₄ and R₅ are defined as in one of claims 1, 2 and 3, characterized in that a product of general formula: 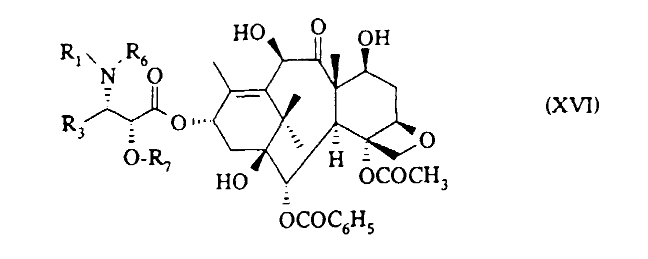 in which R₁, R₃, R₆ and R₇ are defined as in one of claims 1, 2, 3 and 4, is treated by means of a product of general formula:
(R)₃Si-Hal (X)
in which the symbols R, which may be identical or different, represent an alkyl radical containing 1 to 6 carbon atoms, optionally substituted with a phenyl radical, or a cycloalkyl radical containing 3 to 6 carbon atoms or a phenyl radical, to obtain a product of general formula: 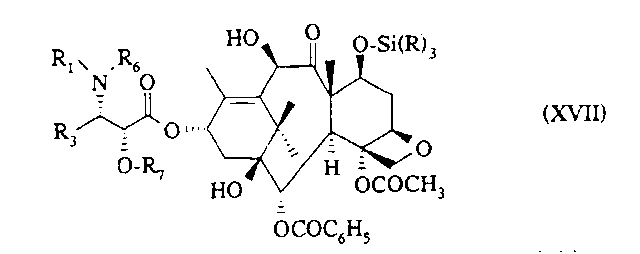 in which R, R₁, R₃, R₆ and R₇ are defined as above, which is functionalized at position 10 by means of a product of general formula:
R'₄-X₁ (XII)
in which R'₄ represents a radical such that R'₄-O is identical to R₄ defined as in one of claims 1, 2 and 3 and X₁ represents a halogen atom or a reactive ester residue, to give a product of general formula: 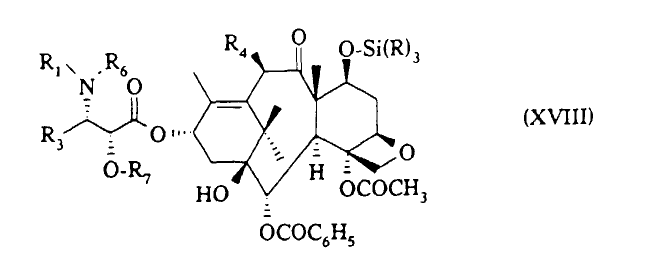 in which R, R₁, R₃, R₄, R₆ and R₇ are defined as above, the silyl protective group of which is replaced by a hydrogen atom to give a product of general formula: 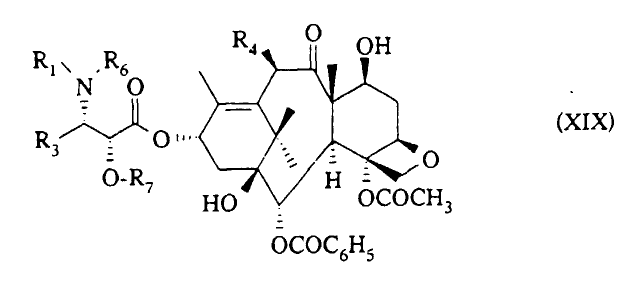 which, by the action of a product of general formula (XV), yields the product of general formula (V), the protective groups of which are replaced by hydrogen atoms to give a product of general formula (I) in which Z represents a radical of general formula (II).

11. Process for preparing a product according to one of claims 1, 2 and 3, characterized in that activated Raney nickel, in the presence of an aliphatic alcohol containing 1 to 3 carbon atoms or an ether, is reacted with a product of general formula: 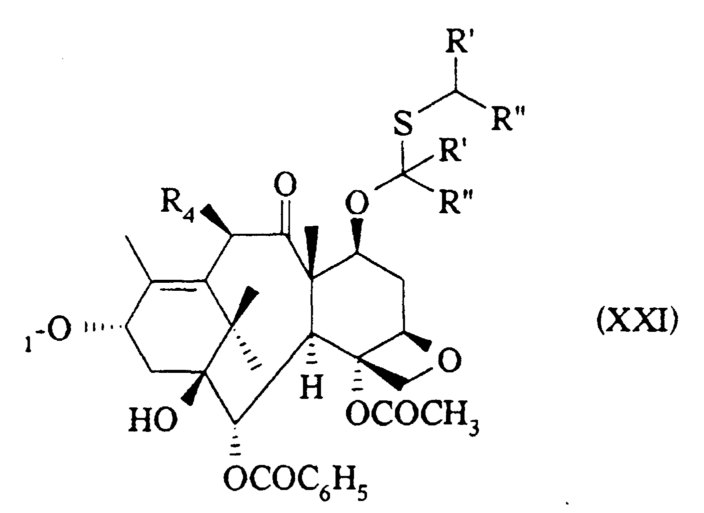 in which R₄ is defined as in one of claims 1, 2 and 3 and R' and R", which may be identical or different, represent a hydrogen atom or an alkyl radical containing 1 to 6 carbon atoms, an alkenyl radical containing 2 to 6 carbon atoms, an alkynyl radical containing 3 to 6 carbon atoms, a cycloalkyl radical containing 2 to 6 carbon atoms or a cycloalkenyl radical containing 3 to 6 carbon atoms, optionally substituted, or alternatively R' and R", together with the carbon atom to which they are linked, form a cycloalkyl radical containing 3 to 6 carbon atoms or a cycloalkenyl radical containing 4 to 6 carbon atoms, and Z₁ represents a hydrogen atom or a radical of general formula: 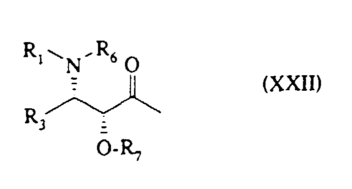 in which R₁ and R₃ are defined as in one of claims 1 to 3 and R₆ and R₇ are defined as in claim 4, to obtain a product of general formula: 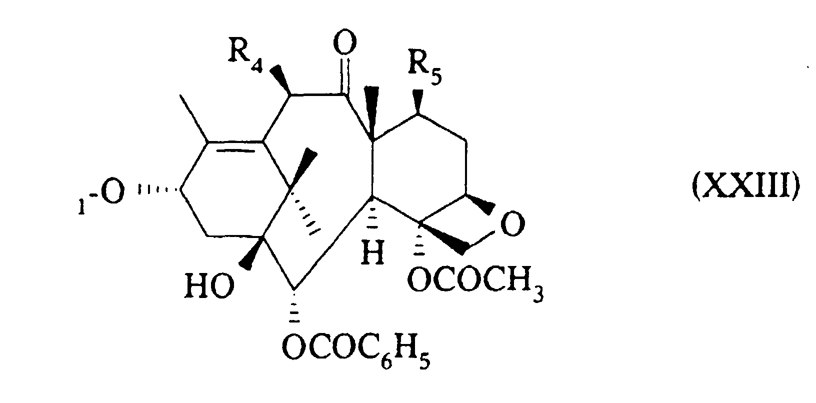 followed, when Z₁ represents a radical of general formula (XXII), by replacement of the protective groups represented by R₆ and/or R₆ and R₇ by hydrogen atoms under the conditions of claim 8.

12. Preparation process according to claim 11, characterized in that it is carried out at a temperature of between -10 and 60°C.

13. 4α-Acetoxy-2α-benzoyloxy-5β,20-epoxy-1β-hydroxy-7β,10β-dimethoxy-9-oxo-11-taxen-13α-yl (2R,3S)-3-tert-butoxycarbonylamino-2-hydroxy-3-phenylpropionate.

14. 4α-Acetoxy-2α-benzoyloxy-1β-hydroxy-5β,20-epoxy-7β-methoxy-10β-ethoxy-9-oxo-11-taxen-13α-yl (2R,3S)-3-tert-butoxycarbonylamino-2-hydroxy-3-phenylpropionate.

15. 4α-Acetoxy-2α-benzoyloxy-1β-hydroxy-5β,20-epoxy-7β-methoxy-10β-(1-propyl)oxy-9-oxo-11-taxen-13α-yl (2R,3S)-3-tert-butoxycarbonylamino-2-hydroxy-3-phenylpropionate.

16. Process for preparing the compound according to claim 13, characterized in that,
in a first step, dimethyl sulphoxide is reacted with 4α-acetoxy-2α-benzoyloxy-5β,20-epoxy-1β,7β,10β-trihydroxy-9-oxo-11-taxen-13α-yl (2R,4S,5R)-3-tert-butoxycarbonyl-2-(4-methoxyphenyl)-4-phenyl-1,3-oxazolidine-5-carboxylate,
in a second step, an ethanolic suspension of Raney nickel is reacted with the product of the first step consisting of 4α-acetoxy-2α-benzoyloxy-5β,20-epoxy-1β-hydroxy-7β,10β-bis(methylthiomethoxy)-9-oxo-11-taxen-13α-yl (2R,4S,5R)-3-tert-butoxycarbonyl-2-(4-methoxyphenyl)-4-phenyl-1,3-oxazolidine-5-carboxylate,
in a third step, an ethanolic solution of hydrochloric acid is reacted with the product of the second step consisting of 4α-acetoxy-2α-benzoyloxy-5β,20-epoxy-1β-hydroxy-7β,10β-dimethoxy-9-oxo-11-taxen-13α-yl (2R,4S,5R)-3-tert-butoxycarbonyl-2-(4-methoxyphenyl)-4-phenyl-1,3-oxazolidine-5-carboxylate.

17. 4α-Acetoxy-2α-benzoyloxy-5β,20-epoxy-1β-hydroxy-7β,10β-bis(methylthiomethoxy)-9-oxo-11-taxen-13α-yl (2R,4S,5R)-3-tert-butoxycarbonyl-2-(4-methoxyphenyl)-4-phenyl-1,3-oxazolidine-5-carboxylate.

18. Pharmaceutical composition, characterized in that it contains at least one product according to one of claims 1, 2 and 3 for which Z represents a radical of general formula (II) and 13 to 15, in combination with one or more pharmaceutically acceptable diluents or adjuvants and optionally one or more compatible and pharmacologically active compounds.

19. Pharmaceutical composition, characterized in that it contains at least the product according to claim 13 in combination with one or more pharmaceutically acceptable diluents or adjuvants and optionally one or more compatible and pharmacologically active compounds.

20. Pharmaceutical composition, characterized in that it contains at least the product according to claim 14 in combination with one or more pharmaceutically acceptable diluents or adjuvants and optionally one or more compatible and pharmacologically active compounds.

21. Pharmaceutical composition, characterized in that it contains at least the product according to claim 15 in combination with one or more pharmaceutically acceptable diluents or adjuvants and optionally one or more compatible and pharmacologically active compounds.
